(19) Europäisches Patentamt
European Patent Office
Office européen des brevets

(11) **EP 1 584 289 B1**

(12) **EUROPÄISCHE PATENTSCHRIFT**

(45) Veröffentlichungstag und Bekanntmachung des Hinweises auf die Patenterteilung:
**06.01.2010 Patentblatt 2010/01**

(51) Int Cl.:
*A61B 5/026* (2006.01)     *A61B 5/022* (2006.01)
*A61B 5/024* (2006.01)     *A61B 5/00* (2006.01)

(21) Anmeldenummer: **05004826.3**

(22) Anmeldetag: **04.03.2005**

(54) **Arbeitsverfahren zum Betreiben einer Vorrichtung und Auswertung von Messergebnissen zur nichtinvasiven Ermittlung hämodynamischer Funktionen einschließlich der endothelialen Funktion**

Device operating method for non-invasive assessing of hemodynamic functions including endothelial status

Procédé d'exploitation pour un appareil permettant l'évaluation non-invasive des fonctions hemodynamiques, y compris celles de l'endothélium

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **17.03.2004   DE 102004013095**
**02.04.2004   DE 102004016376**

(43) Veröffentlichungstag der Anmeldung:
**12.10.2005   Patentblatt 2005/41**

(73) Patentinhaber: **Software + Systeme Erfurt GmbH**
**99198 Erfurt-Kerspleben (DE)**

(72) Erfinder:
• **Grohmann, Gerald, Dr.**
**07743 Jena (DE)**
• **Krauss, Manfred, Prof. Dr.-Ing. habil**
**09127 Chemnitz (DE)**
• **Mandler, Dietrich, Dr.**
**99102 Rohda Niedernissa (DE)**
• **Tietz, Uwe-Jens**
**99102 Klettbach (DE)**

(74) Vertreter: **Kruspig, Volkmar et al**
**Meissner, Bolte & Partner GbR**
**Postfach 86 06 24**
**81633 München (DE)**

(56) Entgegenhaltungen:
**WO-A-02/34105**

• **KELM MALTE: "Flow-mediated dilatation in human circulation: diagnostic and therapeutic aspects." AMERICAN JOURNAL OF PHYSIOLOGY. HEART AND CIRCULATORY PHYSIOLOGY. JAN 2002, Bd. 282, Nr. 1, Januar 2002 (2002-01), Seiten H1-H5, XP002339462 ISSN: 0363-6135**
• **KELM M ET AL: "The nitric oxide/superoxide assay. Insights into the biological chemistry of the NO/O-2. interaction." THE JOURNAL OF BIOLOGICAL CHEMISTRY. 11 APR 1997, Bd. 272, Nr. 15, 11. April 1997 (1997-04-11), Seiten 9922-9932, XP002339463 ISSN: 0021-9258**
• **G. GROHMAN ET. AL.: "Zur Makro- und Mikrozirkulation am Vorfuss unter verschiedenen Kompressionsdrücken bei gesunden Probandinnen" PHLEBOLOGIE, Bd. 29, Nr. 5, Oktober 2000 (2000-10), Seiten 114-123, XP002339464**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Arbeitsverfahren zum Betreiben einer Vorrichtung und Auswerten von Messergebnissen zur nichtinvasiven Ermittlung hämodynamischer Funktionen. Die messtechnische Erfassung und damit die Erkennung von frühen arteriosklerotischen Veränderungen im gesamten arteriellen Herz-KreislaufSystem ist zu einer wesentlichen Aufgabe der klinischen Herz-Kreislauf-Forschung geworden. Auch für die Langzeitbehandlung von chronischen Erkrankungen, wie Diabetes mellitus und Herz-Kreislauf-Erkrankungen insgesamt, gewinnt die Vermeidung von Gefäßwandschäden, vor allem die der "Innenauskleidung" der Gefäße, des Endothels, stark an Bedeutung. Eine Fehlfunktion des "multifunktionellen Organs" Endothel bis hin zur sogenannten "endothelialen Dysfunktion" führt zu einer immer stärkeren Freisetzung von gefäßverengenden Substanzen und zur Bildung freier Sauerstoffradikale [Drexler H, Hornig B: Endothelial dysfunction in human disease. J Mol Cell Cardiol. 1999 Jan;31(1): 51-60]. Allgemein ausgedrückt spielt das Blutgefäß-Endothel eine Schlüsselrolle bei der Entwicklung atherosklerotischer Plaques, bei der Entstehung der koronaren Herzkrankheit sowie vaskulärer Erkrankungen insgesamt. Zur Bestimmung von (peripheren) Gefäßwandeigenschaften einschließlich der endothelialen Dysfunktion existiert bisher kein Meßverfahren, das für eine einfache und breite Anwendung geeignet erscheint.

In überkreuzten Studien, wie von *Schmidt-Trucksäss* und *Huonker* dargelegt wird [*Schmidt-Trucksäss A and M Huonker*: Assessment of arteriosclerotic changes in the carotic artery with noninvasive ultrasound. Z. Kardiol 89: Suppl II/124-II/129 (2000)], konnte generell gezeigt werden, dass eine erhöhte Wandsteifheit in den Carotiden mit verschiedenen kardiovaskulären Risikofaktoren, besonders mit einer ausgedehnten koronaren Herzkrankheit, verbunden ist. Die frühestmögliche Messung der Arterien-Steifheit infolge der Arteriosklerose, also bevor sichtbare Veränderungen der Wandeigenschaften sich ergeben, ist nach *Schmidt-Trucksäss* und *Huonker* jedoch komplizierter als die Messung der Wanddicke.

Gegenwärtig sind die genauesten Methoden zur Bestimmung des lokalen arteriellen Durchmessers und damit auch der Wandsteifheit die Echountersuchung und Mehrfenster-Doppler-Verfahren, jedoch erfordern beide eine Spezialausrüstung und ein spezielles Training. Eine sehr interessante und einfache Methode zur Bestimmung der Wandsteifheit mit nichtinvasivem Ultraschall in der Karotisregion ist die Messung mit Gewebedoppler, wie erstmals von *Schmidt-Trucksäss* und *Huonker* gezeigt wurde.

Insbesondere ist die Bestimmung der endothelialen Dysfunktion Hauptursache nachfolgender Diabetes-Komplikationen (u. a. Herz- und Gefäßerkrankungen bis hin zum Myokardinfarkt bzw. periphere arterielle Verschlußkrankheit mit dem Endpunkt Amputation, Nierenversagen, zerebrovaskuläre Insuffizienz mit dem Endpunkt zerebrovaskulärer Insult), jedoch auch für die *Präeklampsie* als eine Endothelerkrankung, die zu den für Mutter und Kind gefährlichsten Schwangerschaftskomplikationen zählt [Beinder, E. und W. Frobenius: Die Präeklampsie: Eine Endothelerkrankung. Deutsches Ärzteblatt Jg. 97, Heft 41, 13.Oktober 2000], derzeit nur mit speziellen hochauflösenden Ultraschallgeräten und entsprechend ausgebildetem Personal möglich, also kaum im medizinischen Alltag einsetzbar.

Angewendet wird die Methode nach *Celermajer,* die in einer Messung des Durchmessers der Arteria brachialis (Oberarmarterie) vor und nach 10-minütiger suprasystolischer Stauung mit 250 mm Hg Staudruck am Unterarm besteht [Celermajer DS, Sorensen KE, Gooch VM, Spiegelhalter DJ, Miller OI, Sullivan ID, Lloyd JK, Deanfield JE: Non-invasive detection of endothelial dysfunction in children and adults at risk of atherosclerosis. Lancet. 1992;340(8828):1111-5, Celermajer DS: Testing endothelial function using ultrasound. J Cardiovasc Pharmacol. 1998;32 Suppl 3:S29-32.]. Obwohl die Genauigkeit in durchgeführten Studien für die Anwendung in der Klinik als ausreichend befunden wurde [Sorensen KE, Celermajer DS, Spiegelhalter DJ, Georgakopoulos D, Robinson J, Thomas O, Deanfield JE: Non-invasive measurement of human endothelium dependent arterial responses: accuracy and reproducibility. Br Heart J. 1995;74: 247-53], dürften im praktischen medizinischen Alltag erhebliche Fehlermöglichkeiten auftreten. Zur Bestimmung explizit einer Endotheldysfunktion ist außerdem eine Wiederholung der Prozedur mit Gabe eines Vasodilatators notwendig, um eine Gefäßwandstarre als Ursache einer evtl. fehlenden Vasodilatation auszuschließen. Schließlich kann mit dieser Methode nur indirekt auf die Wirkung des vom Blutgefäßendothel mehr oder weniger freigesetzten Gefäßdilatators Stickstoffmonoxid (NO) [Furchgott, R.F. et J. V. Zawadski: The obligatory role of endothelial cells in the relaxation of arterial smooth muscle by acetlycholine. Nature 1980; 288: 373-376; Scriba, P. C. und S. Endres: NO. Mittler, Missetäter und Medikament. Sonderheft Internist 1997. 38:405 恬 Springer 1997; Schmidt, H.H.H.W.: NO, endogener Botenstoff und Zellgift. MED. MO. PHARM. 17, 6 (1994), 168 - 185; Kelm, M.: Kardiovaskuläre Wirkungen von Stickstoffmonoxid und ihre Bedeutung für die arterielle Hypertonie. Schattauer Stuttgart/New York 1996] geschlossen werden. Wegen der kostenintensiven Gerätetechnik mit ihren Limitierungen [Corretti MC, Anderson TJ, Benjamin EJ, Celermajer D, Charbonneau F, Creager MA, Deanfield J, Drexler H, Gerhard-Herman M, Herrington D, Vallance P, Vita J, Vogel R: Guidelines for the ultrasound assessment of endothelial-dependent flow-mediated vasodilation of the brachial artery: a report of the International Brachial Artery Reactivity Task Force. J Am Coll Cardiol. 2002;39:257-65], des zeitlichen Aufwandes (zweimalige Durchführung) und der notwendigen praktischen Erfahrung wird dieses Verfahren nicht breit bzw. kaum für ein Screening in klinischen Praxen anwendbar sein.

Es ist daher Aufgabe der Erfindung, mit Hilfe der bekannten nichtinvasiven **N**ahen-**I**nfrarot-Rot-**R**emissions-**P**hotoplethysmographie - der **NIRP-Methode** - periphere Gefäßwandeigenschaften einschließlich der endothelialen Dysfunktion unter Anwendung einer geeigneten Vorrichtung zu bestimmen.

Als geeignete Vorrichtung wird in diesem Zusammenhang eine Vorrichtung zur nichtinvasiven Ermittlung hämodynamischer Funktionen des Blutgefäßsystems nach WO 02/314105 A2 [Method and apparatus for non-invasively evaluating endothelial activity in a patient] erachtet. In der Offenlegungsschrift werden Mittel zum Anlegen einer Blutdruck-Staumanschette an den Extremitäten eines Individuums und gleichzeitige nichtinvasive Ableitung der an der entsprechenden Gefäßperipherie sich einstellenden mikrovaskulären Blutvolumenpulsation offenbart. Zudem sind aus der Schrift Mittel zur Erzeugung einer Stauung bis zu einem Kompressionsdruck bekannt, bei dem die an der Gefäßperipherie dieser Extremität abgeleiteten Blutvolumenpulsationen nicht mehr ableitbar sind. Außerdem werden Mittel zum sprungartigen Öffnen der Kompression nach einer Stauzeit von 5 ... 10 Minuten und Mittel für allgemeine Funktionstests, die zu Veränderungen vor allem der Schubspannung des strömenden Blutes auf eine Gefäßwand führen und damit durch spezielle externe Verfahren bzw. Stimuli, auch unter Pharmaka, gezielt Einfluss auf eine Freisetzung des Vermittlers einer endothelvermittelten Kreislaufregulation, von Stickstoffmonoxid (NO), genommen wird, benötigt. Die beschriebenen und bekannten Mittel finden Verwendung in dem nachfolgend beschriebenen Arbeitsverfahren zum Betreiben der bekannten Vorrichtung und zur Auswertung von Messergebnissen zur nichtinvasiven Ermittlung hämodynamischer Funktionen des Blutgefäßsystems, einschließlich des Endothels.

Aus der Physiologie ist bekannt [Thews, G. und P. Vaupel: Vegetative Physiologie. 3. Aufl. Springer Berlin/ Heidelberg/New York/Barcelona/Budapest/ Hongkong/ London/ Mailand/Paris/Santa Clara/Singapur/Tokio 1997], dass in den Blutgefäßwänden der Anteil an *elastischen Fasern* relativ groß ist. Dabei verhält sich eine Arterie vergleichsweise wie ein Gartenschlauch, dessen innere Schichten aus dehnbarem Gummi von einer stabilisierenden Leinenhülle umgeben sind [Burton, A.C.: Physiologie und Biophysik des Kreislaufs. F.K. Schättauer Stuttgart/ NewYork 1969]. Der dehnbare Gummi wiederum ist vergleichbar mit den elastischen Fasern der Blutgefäße, die in allen Gefäßarten (außer Kapillaren und arteriovenösen Anastomosen) reichlich vorhanden sind. Unmittelbar unter dem Endothel liegend und damit dazu in ständiger Wechselwirkung bilden sie eine Schicht, die elastische Intima. Sie treten auch z. T. in der Media und Adventitia auf. Das Charakteristikum dieser elastischen Fasern besteht in der leichten Dehnbarkeit (nach *Burton* etwa 6mal leichter als Gummi). Ehe sie ihre Elastizitätsgrenze erreichen, können sie um das Vielfache ihrer ursprünglichen Länge gedehnt werden. Nach *Burton* besteht die Aufgabe dieser Fasern darin, automatisch eine elastische Spannung auszuüben und damit, ohne Aufwand von biochemischer Energie, der dehnenden Kraft des Blutdruckes einen Widerstand entgegenzusetzen, auch wenn sie sich diese Aufgabe mit den in der Media und Adventitia liegenden kollagenen Fasern (entspricht der stabilisierenden Leinenhülle des Gartenschlauches) teilen. Letztere stellen jedoch einer Dehnung viel mehr Widerstand entgegen als elastische Fasern. Nach *Burton* ist deren Elastizitätsmodul etwa 100mal größer als der von elastischen Fasern, d.h. eine relativ kleine Anzahl kollagener Fasern in einer Arterienwand bewirkt einen hohen Dehnungswiderstand. Sie können folglich erst dann einen Gegendruck ausüben, wenn die Gefäßwand bis zu einem gewissen Grad gedehnt ist. Wenn das Ausmaß der Dehnung allmählich zunimmt, erreichen immer mehr dieser steiferen Fasern ihre volle Länge und widersetzen sich dann einer weiteren Streckung. Allgemein gilt nach Burton, dass Blutgefäße der Dehnung umso mehr Widerstand leisten, je mehr sie gedehnt sind.

Bekanntlich wird bei zunehmendem Füllungsdruck das Lumen der Gefäße erweitert, bei nachlassendem Druck geht es wieder in den Anfangszustand zurück. Des weiteren ist bekannt, dass mit jeder Systole des Herzens ein Blutvolumen von ca. 70 - 140 ml in das arterielle Blutgefäßsystem ausgeworfen wird. Im Anfangsteil der Aorta führt diese Volumenzunahme nach *Thews* zu einem lokalen Druckanstieg und damit zu einer elastischen Erweiterung dieses Gefäßabschnittes, so dass ein Teil des ausgeworfenen Volumens kurzzeitig gespeichert wird.

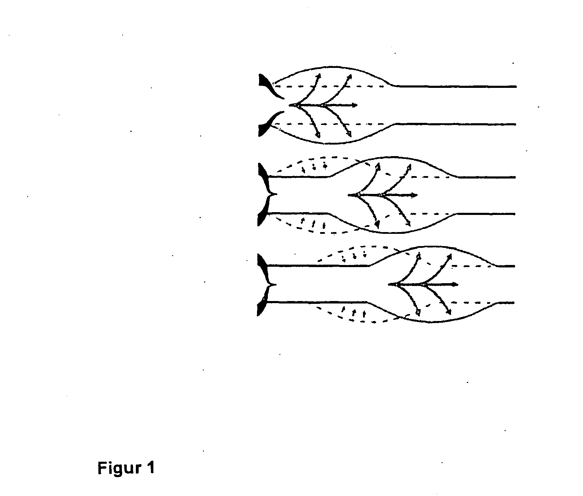

*Thews* legt dar, dass auf Grund des lokal erhöhten Druckes das jeweils gespeicherte Blut in den nächsten Gefäßabschnitt strömt, wo wiederum unter zunehmendem Druck der Gefäßquerschnitt erweitert wird (Figur 1). Schließlich setzt sich dieser Vorgang kontinuierlich über das gesamte arterielle Gefäßsystem fort. Wegen der elastischen Dehnbarkeit der arteriellen Gefäßwände entstehen ebenfalls nach *Thews* "Pulswellen", die mit einer bestimmten Geschwindigkeit über das arterielle Gefäßsystem laufen. Mit der bekannten nichtinvasiven NIRP-Methode sind die dabei auftretenden "Volumenpulse" als "mikrovaskuläre Blutvolumenpulsationen" an einem peripheren Messort ableitbar. Folglich muss es erfindungsgemäß möglich sein, aus der Form dieser mikrovaskulären Volumenpulsationen ein Maß für die elastische Faser-Dehnung und weitere daraus folgende Zusammenhänge - auf Grund der unmittelbaren Entfernung auch zum Gefäßendothel - abzuleiten.

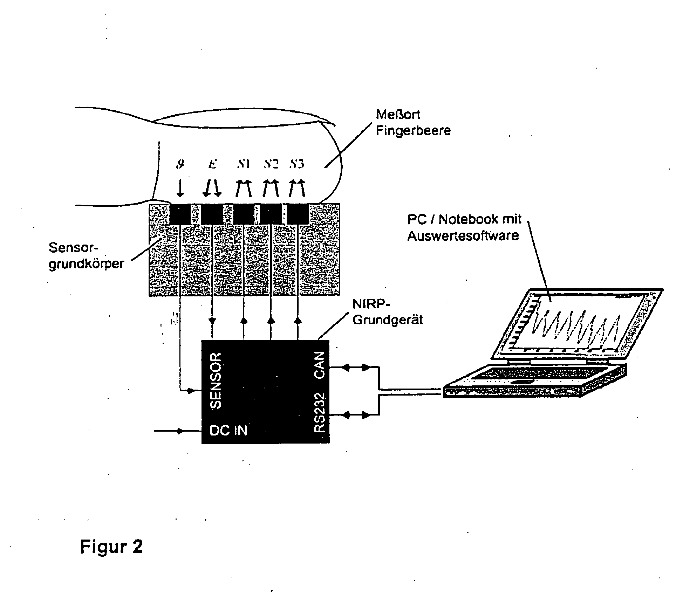

Bei der nichtinvasiven *NIRP-Methode* wird ein Clip, analog zum bekannten Pulsoxymeter, an einem peripheren Meßort eines liegenden Patienten angebracht (vorzugsweise Finger- bzw. Großzehenbeere). Wird nun Licht des Nahen-Infrarot-Bereiches der Wellenlänge von etwa S1 = 840 nm in das Gewebe eingeleitet, so werden relativ große Gewebevolumina bei Eindringtiefen von ca. 3...4 mm illuminiert. Die entstehenden Rückstreusignale werden über einen Empfänger E erfaßt und diagnostisch bewertet. Es lassen sich mit diesem Licht die am speziellen Meßort je Zeiteinheit passierenden (relativen) mikrovaskulären Mengen des Blutes, ein mikrovaskulärer Blutvolumenfluß, ableiten, woraus neben Bewertungen der "Durchblutung" auch gleichzeitig die des kardiovaskulären Zustandes möglich ist. Mit der Rot-Wellenlänge von etwa S2 = 640 nm kann die derart erfaßte mikrovaskuläre Blutmenge hinsichtlich ihrer Sauerstoffsättigung sowie ihrer Veränderung (z. B. unter einer Therapie mit Pharmaka bzw. auch unter einer physikalischen Therapie) beurteilt werden [Europäische Patentanmeldung Nr. 97118971.7: Vorrichtung und Arbeitsverfahren zum Betreiben der Vorrichtung zur nichtinvasiven Messung und Auswertung von Kreislauf-Kennfunktionen und Parametern]. Zur Kontrolle der Temperatur am Meßort ist ein entsprechender Sensor im Clip integriert. Figur 2 zeigt das prinzipielle Verfahren.

Wie noch zu darzustellen ist, lassen sich mit den Wellenlängen S1 und S2 *indirekt* Stickstoffmonoxid(NO)-vermittelte Folgereaktionen in Echtzeit erfassen, da eine erhöhte NO-Freisetzung zu einer peripheren Gefäßdilatation in Verbindung mit einer erhöhten NO-Oxydierung und dadurch zu einer pulsatilen $O_2$-Reduzierung am Messort führt. Eine Erweiterung der NIRP-Methode mit einer zusätzlichen "NO-Wellenlänge" S3 soll hierfür den Lösungsansatz darstellen, wie nachfolgend erfindungsgemäß dargestellt wird.

Zur kontinuierlichen Erfassung von NO und den sich daraus ergebenden Folgereaktionen werden die Ergebnisse des differenzspektrophotometrischen Messprinzips, wie sie von *Kelm* [Kelm, M.: Kardiovaskuläre Wirkungen von Stickstoffmonoxid und ihre Bedeutung für die arterielle Hypertonie. Schattauer Stuttgart/New York 1996] begründet werden, den weiteren Betrachtungen zugrunde gelegt. Demnach stellt die Wellenlänge von 411 nm einen isosbestschen Punkt dar, wo die Absorptionscharakteristika von Oxyhämoglobin und Methämoglobin identisch sind, dagegen bei 401 nm die größte positive Differenz zwischen den Absorptionsspektren von Oxy- und Methämoglobin auftritt. Aus der zunehmenden Absorptionsdifferenz von 401 vs. 411 nm kann die Zunahme der Konzentration an Methämoglobin und damit auch an NO errechnet werden. Bezogen auf die NIRP-Methode lässt sich folglich mit der Wellenlänge von ca. 401 nm eine Pulsation je Herzaktion analog zum mikrovaskulären Blutvolumen bei 840 nm ableiten und - entsprechend zur bekannten Sauerstoffsättigungsmessung mittels Pulsoxymeter - eine *relative* (keine absolute) NO-Veränderung ermitteln. Dies ist zur Bestimmung des endothelialen Verhaltens und für die medizinische Praxis ausreichend. Außerdem ist es dadurch möglich, die sich unter einer Therapie einstellenden relativen NO-Veränderungen abzuleiten. Aus dem von *Kelm* [Kelm, M.: Kardiovaskuläre Wirkungen von Stickstoffmonoxid und ihre Bedeutung für die arterielle Hypertonie. Schattauer Stuttgart/New York 1996] ermittelten Differenzspektrum von Oxyhämoglobin vs. Methämoglobin kann zur NO-Bewertung der Wellenlängenbereich von 390...405 bzw. 415...430 nm zugrunde gelegt werden, wobei 411 nm als isosbestscher Punkt ermittelt wurde.

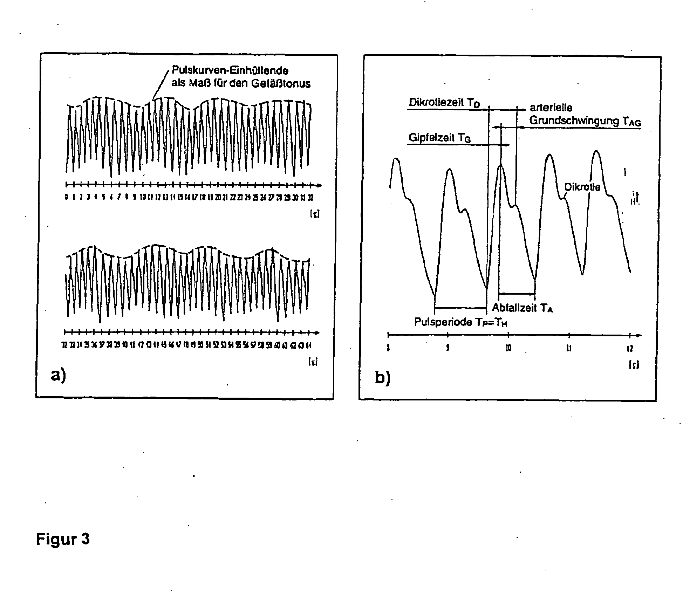

In Figur 3a ist als Beispiel eine nach der nichtinvasiven NIRP-Methode gemessene Blutvolumenpulsation im Mikrogefäßbereich "Fingerbeere" bei einem 25jährigen liegenden Normalprobanden unter Ruheventilation dargestellt (Meßzeit 64 Sekunden). Dabei ist den Pulsationen ein Signal überlagert, das dem Gefäßtonus (Mikrozirkulation, Sympathikusaktivität, Atmungseinfluß) entspricht. Sind der Sympathikuseinfluß oder die Atmungskomponente erhöht, so ist der Gefäßtonus groß. Figur 3b zeigt die in Figur 3a ersichtlichen Volumenpulsationen in einem 4-Sekunden-Zeitfenster mit charakteristischen Zeitparametern, gleichfalls mit der Pulsperiode einer zugehörigen Herzaktion. In [*Grohmann, G.; Krauß, M. und S. Müller:* Vergleichende Untersuchungen zur autonomen kardialen Neuropathie bei Patienten mit Diabetes mellitus mit dem NIRP- und dem ProSciCard-Verfahren. PERFUSION 12; 1999: 392-408.5] wird gezeigt, dass diese Pulsperiodendauer mit der ursächlichen Herzperiodendauer bei in den meisten Fällen der klinischen Praxis ausreichender Genauigkeit mit dem RR-Abstand im EKG und damit mit den bekannten kardiovaskulären Kennwerten und - funktionen übereinstimmt.

Die im abfallenden Volumenpuls nach Figur 3b deutlich ausgeprägte Dikrotie und deren Lage in etwa 2/3 der Gesamthöhe dieses Pulses weisen auf ein "normales" arterielles System bis zum Meßort hin. Dabei ist eine Dikrotie Ausdruck einer im arteriellen System auftretenden mehr oder weniger gedämpften Schwingung des Blutvolumens.

Eine stark ausgebildete Dikrotie ist ein zuverlässiges Kennzeichen eines leistungsfähigen und gut regulierbaren Arteriensystems. So ist eine solche Dikrotie besonders markant bei jungen Menschen ausgeprägt, speziell bei Hochleistungssportlern. Ebenfalls ist bekannt, dass sich bei einer Gefäß-Dilatation eine auftretende dikrote Welle verstärkt und der Basis nähert. Umgekehrt bedeutet ein Höherwandern der Dikrotie mit Abflachung der am abfallenden Pulsschenkel auftretenden Schwingung eine arterielle Engerstellung, so dass eine Dikrotie oft nicht mehr ersichtlich ist.

Offensichtlich läßt sich aus der *Form* dieser durch das entsprechende arterielle Gefäßsystem geprägten Volumenpulsation *qualitativ* auf dessen Zustand schließen. In mehreren Arbeiten, insbesondere Deutsches Patent Nr. 42 38 641 [Vorrichtung und Arbeitsverfahren zum Bestimmen und Auswerten des physiologischen Zustandes von Gefäßsystemen] sowie Deutsche Patentanmeldung P 43 22 860.7-35 [Verfahren und Vorrichtung zur Bestimmung und Auswertung des Zustandes von Gefäßsystemen], wird gezeigt, dass ebenso eine quantitative Bewertung möglich ist. Ist z.B. die sogenannte "arterielle Grundschwingung" $T_{AG}$ (s. Figur 3b) erloschen (damit keine Dikrotie ersichtlich), so ist dies ein sicheres Zeichen einer Schädigung des Arteriensystems bzw. ist der Verlust der Dikrotie Hinweis auf eine gewisse Regulationsstarre.

Dann kann z. B. nach der deutschen Patentanmeldung P 43 22 860.7-35 [Verfahren und Vorrichtung zur Bestimmung und Auswertung des Zustandes von Gefäßsystemen] kein *Relativer Peripherer Widerstand* $RPW_2$ abgeleitet werden. Als Charakteristika für das Auftreten einer *peripheren arteriellen Verschlußkrankheit (pAVK)* gilt sowohl das Erreichen eines späten und kuppelförmigen Gipfels als auch ein nachfolgender etwa linearer Abfall, während sich eine mediasklerotische Gefäßveränderung ebenfalls durch einen linearen Abfall nach Erreichen des Blutvolumengipfels darstellt, jedoch der Gipfel nahezu spitzförmig ist. Auch hierfür ist wegen fehlender Dikrotien kein derartiges peripheres Widerstandsmaß zuordenbar.

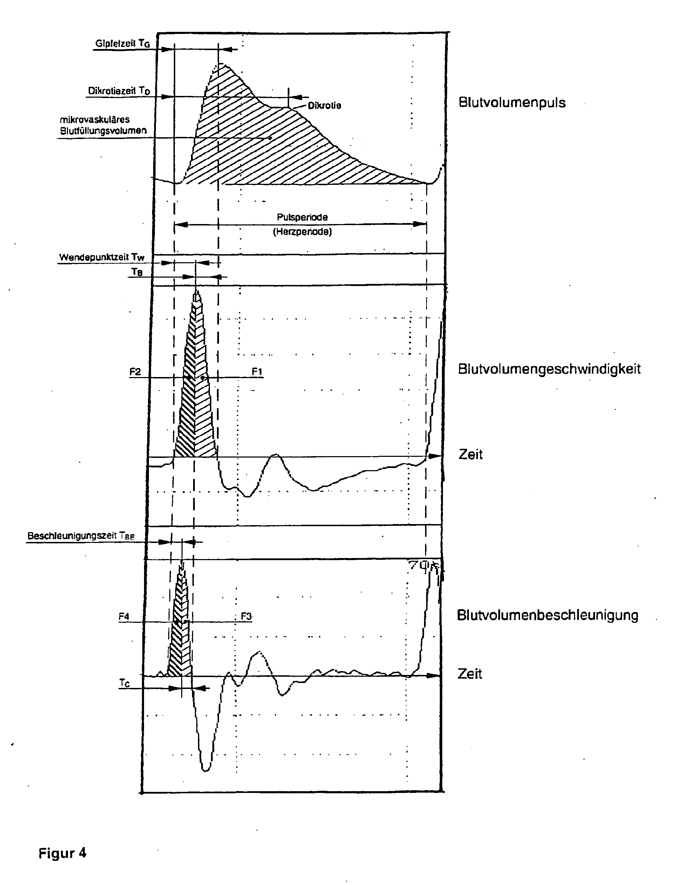

Erfindungsgemäß werden nun aus den pulsierenden mikrovaskulären Blutvolumina mathematische Ableitungen (Differentiationen) gebildet, wie aus Figur 4 hervorgeht. Die erste Ableitung stellt die *Blutvolumengeschwindigkeit dar*, die zweite die zugehörige *Blutvolumenbeschleunigung.* Grundsätzlich stellt die peripher gemessene Blutpulsation eine Funktion des aktuell im illuminierten Bereich befindlichen Blutvolumens und der in diesem Blutvolumen enthaltenen Konzentration eines zu bestimmenden Stoffes dar, wobei das Meßsignal dominant von dem illuminierten Blutvolumen bestimmt wird, so dass es im physikalischmathematischen Sinne möglich ist, die entsprechenden Ableitungen eines Blutvolumens zu bilden. Die Abhängigkeit des Meßsignals von der Konzentration eines Stoffes erlaubt im Umkehrschluß die Ermittlung der Konzentration dieses Stoffes sowie die Messung der Dynamik der Konzentration über der Zeit.

Es werden für eine Pulsperiode folgende Verhältnisse erfindungsgemäß abgeleitet:

- Nach Durchlaufen des Pulskurvenminimums steigt ein mikrovaskuläres Blutvolumen kontinuierlich bis zum Erreichen des Pulsgipfels (Gipfelzeit $T_G$) an. Es erhöht sich hierbei die entsprechende Blutvolumengeschwindigkeit bis zur Wendepunktzeit $T_W$, wo stets das *Maximum innerhalb einer Pulsperiode* auftritt. *Eine im Vergleich zu einem Normalbereich erhöhte Wendepunktzeit $T_W$, so wurde erkannt, deutet auf eine arterielle Durchblutungsverringerung im weitesten Sinne hin.* Damit stellt sich $T_W$ als arterielles *Gütemaß dar.*

- Da nach Erreichen von $T_W$ eine Blutvolumenpulsation nur noch langsamer ansteigt, reduziert sich danach die zugehörige Blutvolumengeschwindigkeit kontinuierlich, jedoch abhängig vom arteriellen Gefäßzustand.

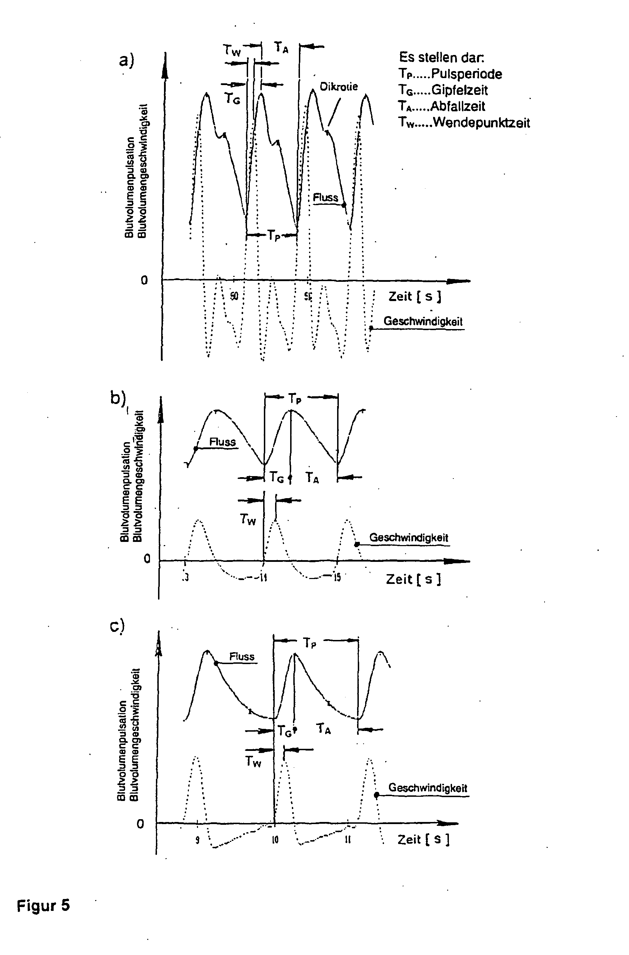

- Bei der Gipfelzeit $T_G$ stellt sich der maximale Blutvolumenpuls ein, so dass in diesem Punkt eine Umkehr von positiver zu negativer Blutvolumengeschwindigkeit erfolgt. Deshalb kann $T_G$ *als Maß für die arterielle Durchblutung* bezeichnet werden. Bei pAVK-Patienten sowie bei Patienten mit Mediasklerose muß im Vergleich zu Normalpersonen (Figur 5a) diese Gipfelzeit erhöht sein (Figuren 5b, c). Ein derartiges Verhalten der Gipfelzeit sowie der Wendepunktzeit bei verringerter Durchblutung lässt sich aus dem Modell des P-Systems mit Verzögerung 2. Ordnung der linearen Systemtheorie bzw. des Schwingkreises der Elektrotechnik ableiten [KrauB, M. und E.-G. Woschni: Meßinformationssysteme. Kennfunktionen, Gütekriterien, Optimierung. 2.Aufl. VEB Verlag Technik 1975]. Hier ist bekannt, dass sich mit größerer Dämpfung - entspricht dem Fall der arteriellen Durchblutungsstörung - die Form der Sprung- bzw. Stoßübergangsfunktion in gleichem Sinne verändert: die Maxima des Überschwingens werden amplitudenmäßig reduziert und nach höheren Zeiten verschoben.

- Tritt eine dikrote Welle im abfallenden Pulsschenkel des Blutvolumenflusses auf, so kann je nach "Güte" der Dikrotie auch die Blutvolumengeschwindigkeit zeitweise wieder positiv werden, *d.h. der Zustand des arteriellen Gefäßsystems bildet sich auch im Verlauf der Blutvolumengeschwindigkeit nach Erreichen ihres Maximalwertes ab, so dass daraus eine qualitative Bewertung möglich wird.* Bei der Auswertung derartiger Verläufe treten deutliche Unterschiede zwischen pAVK und einer mediasklerotischen Gefäßveränderung auf, wie aus Figur 5 hervorgeht.

- Es wurde bereits dargelegt, dass die dikrote Welle im weitesten Sinne eine Schwingung des Blutvolumens darstellt. Ein derartiges Verhalten des arteriellen Kreislaufsystems entspricht näherungsweise wiederum dem eines P-Systems mit Verzögerung 2. Ordnung der Systemtheorie. Ein solches System ist bekanntlich gekennzeichnet durch 2 voneinander unabhängige Energiespeicher: Masse und Feder bzw. Kondensator und Spule. Je nach Dämpfung D (direkt proportional der mechanischen Dämpfung k bzw. dem elektrischen Widerstand R) stellt sich ein schwingendes und aperiodisches Verhalten ein. Letzteres ist vergleichbar mit dem Fall einer arteriellen Durchblutungsstörung.

Figur 5 zeigt charakteristische Verläufe für mikrovaskuläre Blutvolumenpulsationen sowie daraus abgeleitete Blutvolumengeschwindigkeiten einschließlich markante Zeitabschnitte bei charakteristischen Fällen am jeweiligen Messsort Großzehenbeere (Figur 5a Normalproband, Figur 5b Patient mit peripherer arterieller Verschlusskrankheit sowie Figur 5c mit Mediasklerose). Deutlich unterscheidet sich vor allem die Form der Blutvolumengeschwindigkeiten innerhalb einer Pulsperiode, so dass daraus sowohl qualitative als auch quantitative Maße ableitbar sind.

Erfindungsgemäß wurde erkannt, dass im Geschwindigkeitssverlauf der zugehörigen Blutvolumenpulsation entsprechend Figur 4 zwei markante *Flächen $F_1$* und *$F_2$* auftreten. Dabei lässt sich mit ausreichender Näherung für dieses zu kennzeichnende Flächenverhältnis $F_1$ / $F_2$ das Verhältnis der zugehörigen Zeiten $T_B$ / $T_W$ gleichsetzen, d. h.

$$\frac{Fläche\ F_1}{Fläche\ F_2} \approx \frac{T_B}{T_W} = \frac{T_G - T_W}{T_W} = \frac{T_G}{T_W} - 1. \tag{1}$$

Des weiteren wurde erkannt, dass bei Herausbildung dominanter Dikrotien im Blutvolumenpuls, also bei Auftreten einer Gefäßdilatation, die Fläche $F_1$ im Verhältnis zur Fläche $F_2$ entsprechend reduziert erscheint, andererseits bei Auftreten einer pAVK und damit fehlender Dikrotie sich dies umkehrt.

Wie dargestellt wurde, wird mit jeder Systole des Herzens das entsprechende Blutvolumen in das arterielle Blutgefäßsystem ausgeworfen, so dass sich bei zunehmendem Füllungsdruck das Lumen der Gefäße während dieser Zeit erweitert, bei nachlassendem Druck es wieder in den Anfangszustand zurückgeht. Folglich muß sich auf Grund des Zusammenwirkens des Herzens mit der Gefäßperipherie das kardiovaskuläre Zeitintervall "Systolenzeit" nach einer Laufzeit qualitativ in der Gefäßperipherie abbilden, speziell in der Gipfelzeit $T_G$. Eine verlängerte Systolenzeit, wie sie sich bei arterieller Verschlusskrankheit ergibt, korreliert mit einer erhöhten peripheren Gipfelzeit $T_G$ und umgekehrt. Folglich kann die Gipfelzeit $T_G$ mit ausreichender Näherung gleichgesetzt werden mit der Zeit, innerhalb derer die jeweilige (periphere) arterielle Gefäßdehnung erfolgt. Erfindungsgemäß wurde Gl. (1) als Maß für die elastische Faser-Dehnung erkannt. Dieses dimensionslose Maß wird deshalb Faser-Stretching FS genannt:

$$Faser - Stretching \ FS = \frac{Fläche \ F_1}{Fläche \ F_2} \approx \frac{T_B}{T_W} = \frac{T_G - T_W}{T_W} = \frac{T_G}{T_W} - 1 \qquad (2)$$

Ist also die Fläche $F_1$ im Verhältnis zu $F_2$ reduziert (analog das zugehörige Zeit-Verhältnis $T_B/T_W$), so werden die elastischen Fasern wenig gedehnt sein (evtl. im Vergleich zum Normalfall "unterdehnt"), dagegen ist das Faser-Stretching FS bei arteriellen Duchblutungsstörungen auf Grund des erhöhten peripheren Widerstandes, wie erkannt wurde, i. a. größer als normal.

Der in der Deutschen Patentanmeldung P 43 22 860.7-35 [Verfahren und Vorrichtung zur Bestimmung und Auswertung des Zustandes von Gefäßsystemen] als Verhältnis von Dikrotiezeit $T_D$ und arterieller Grundschwingung $T_{AG}$ (s. Figur 3) abgeleitete *Relative Periphere Widerstand RPW$_2$* korreliert, wie erkannt wurde, mit dem eingeführten Faser-Stretching FS. Dies ist bedeutsam, da bei fehlenden Dikrotien kein quantitatives Maß für RPW$_2$ abgeleitet werden kann.

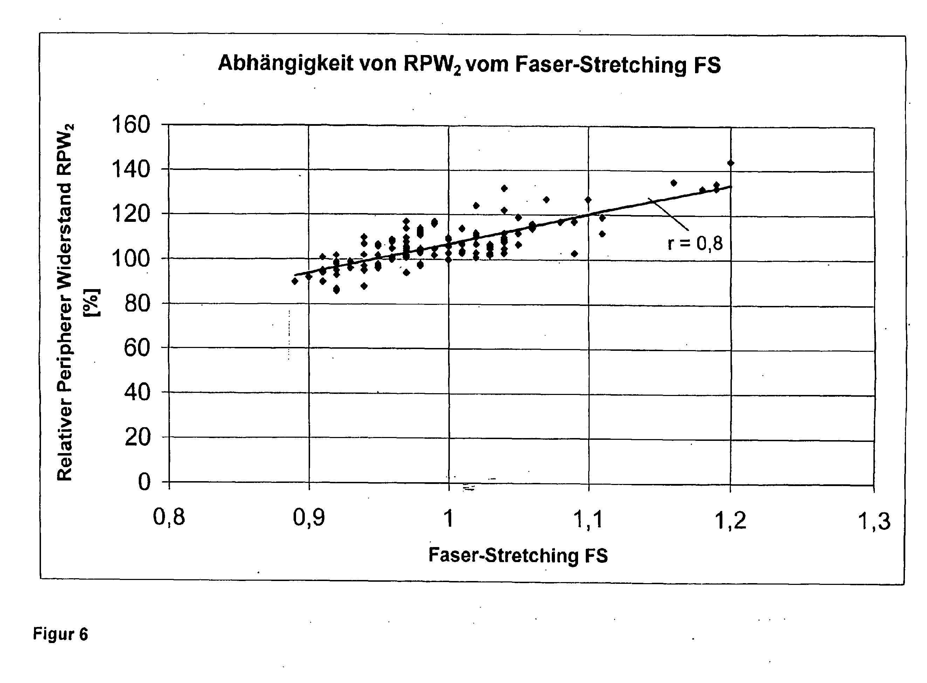

Von 29 Versuchspersonen mit z. T. markant auftretender dikroter Schwingung (Normalpersonen, Leistungssportler, Sichelzellpatienten, Typ I-Diabetiker) wurden sowohl von Finger- als auch Großzehenbeere insgesamt 120 64s-Messungen abgeleitet und sowohl RPW$_2$ als auch FS ermittelt, wie aus Figur 6 hervorgeht. Dabei stellte sich eine Korrelation von *r = 0,8* ein, so dass erfindungsgemäß geschlussfolgert werden kann:

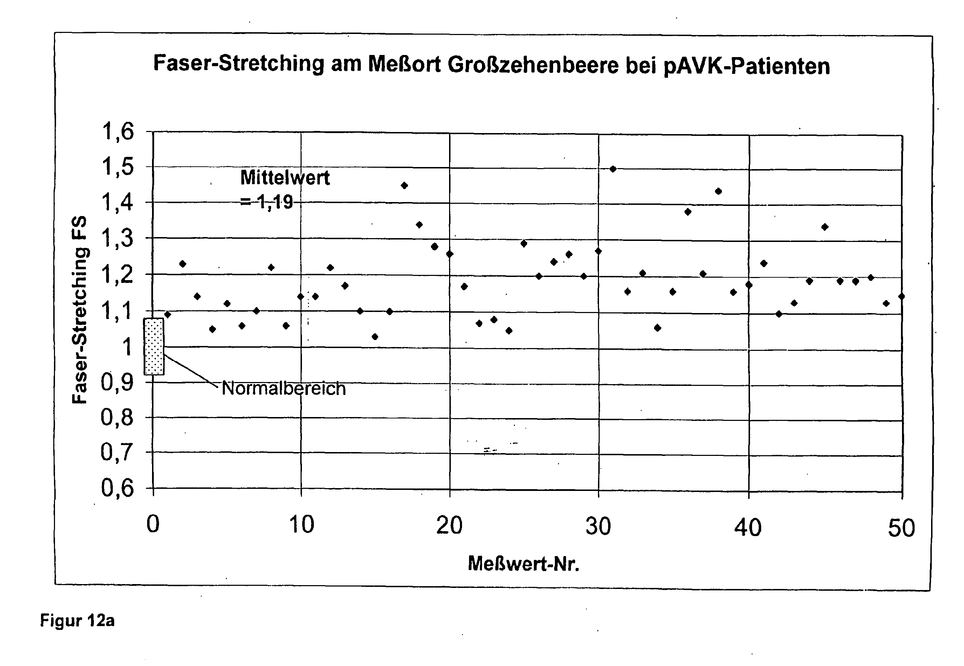

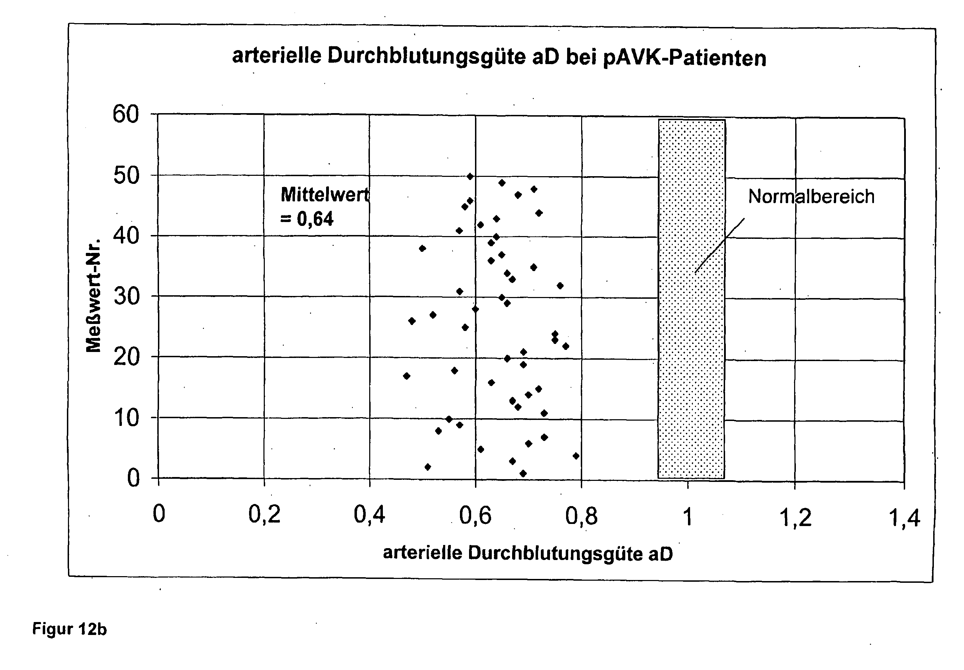

- Der Relative Periphere Widerstand RPW$_2$ ist ein Dehnungsmaß für die elastischen Fasern: Je höher RPW$_2$, desto größer ist deren Dehnung und umgekehrt.
- Näherungsweise kann gesetzt werden: RPW$_2$ ~ FS, d. h. FS ist als Dehnungsmaß einem peripheren Widerstandsmaß gleichsetzbar und demnach auch bei fehlenden Dikrotien, wie sich bei einer endothelialen Dysfunktion meist ergibt, bestimmbar. Dabei ist zu erwarten, wie im Ausführungsbeispiel nach Figur 12 gezeigt wird, dass bei arteriellen Durchblutungsstörungen im weitesten Sinne das Faser-Stretching größer als normal und damit der periphere Widerstand erhöht ist.

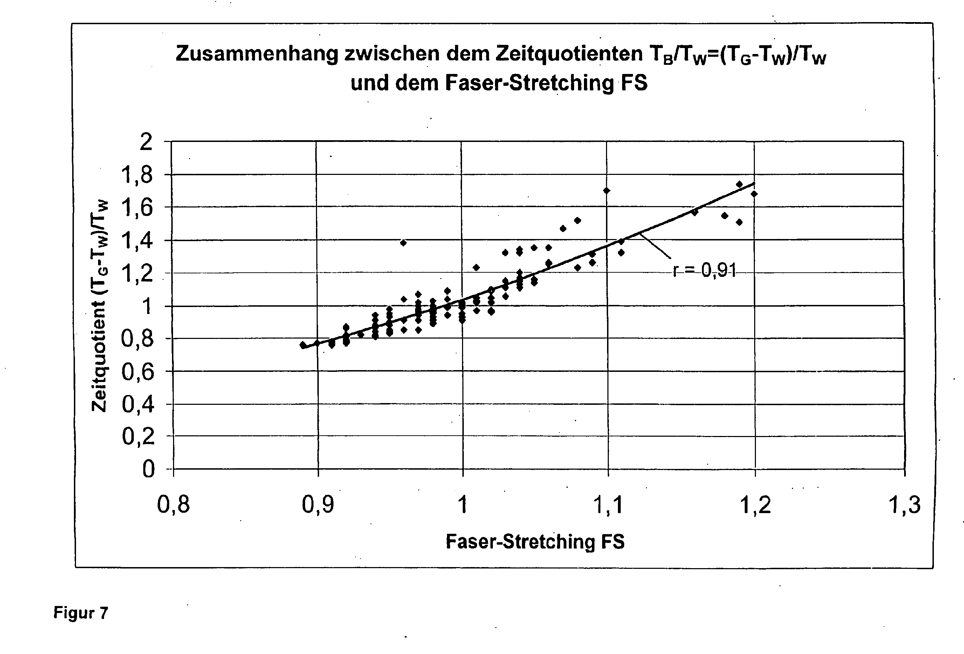

Der in Gl. (2) dargestellte (näherungsweise gültige) Zusammenhang zwischen dem Faser-Stretching FS als Flächenverhältnis $F_1 / F_2$ und dem zugehörigen Zeit-Verhältnis $T_B/T_W = (T_G-T_W)/T_W$ ist in Figur 7 ersichtlich, wo für die 29 Versuchspersonen nach Figur 6 derselbe dargestellt ist. Es ergibt sich eine Korrelation von r = 0,91, so dass die praktische Gültigkeit von Gl. (2) gezeigt ist.

Ermittlung von Normalbereichen der peripheren NIRP-Parameter Von 35 herzkreislaufgesunden liegenden Versuchspersonen (20 weiblich, 15 männlich) mit einem Durchschnittsalter von 29,6 ± 3,9 Jahren wurden in einem ruhigen Raum bei Normaltemperatur sowohl von Finger- als auch Großzehenbeeren insgesamt 70 Blutvolumen-Messungen von jeweils 64 Sekunden Meßdauer abgeleitet. Daraus ergaben sich als *Normalbereiche N* für Faser-Stretching FS, Gipfelzeit $T_G$ und Wendepunktzeit $T_W$ sowie Relativer Peripherer Widerstand RPW$_2$

$$N \ (FS) = 1 \ \pm \ 0,06,$$

$$N \ (T_G) = 213 \ \pm \ 18 \ ms,$$

$$N (T_W) = 101 \pm 7 \text{ ms,}$$

$$N (RPW_2) = 1{,}7 \pm 0{,}08.$$

Ein erhöhtes Faser-Stretching FS wird ebenso wie eine vergrößerte Wendepunktzeit $T_w$ auf eine Durchblutungsverringerung im weitesten Sinne hindeuten, so dass aus diesen Parametern erfindungsgemäß ein arterielles Durchblutungsmaß, "*arterielle Durchblutungsgüte aD*" genannt, bestimmt werden kann:

$$aD \approx \frac{1}{FS \bullet T_W} \qquad\qquad (3)$$

Die Berechtigung einer solchen multiplikativen Verknüpfung ist mathematisch dann gegeben, wenn beide Größen voneinander statistisch unabhängig sind. Aus den obigen 120 Messungen bei den 29 Versuchspersonen mit z. T. markant auftretenden dikroten Schwingungen (Figur 6) erhält man einen Korrelationswert von r = 0,12 (Figur 8), so dass diese Annahme mit ausreichender Näherung erfüllt ist. Bei den pAVK-Patienten nach Figur 9 verringert sich dieser Wert auf r = - 0,01 noch weiter.

aD soll erfindungsgemäß eine dimensionslose und auf den Wert 1 normierte Größe darstellen. Da FS bereits dimensionslos ist und den Mittelwert 1 aufweist, ist lediglich durch Bezug auf den Normalwert von $T_W$, d. h. 101 ms, die quantitative Größe von aD zu bestimmen:

$$aD = \frac{101\,[ms]}{FS \bullet T_W\,[ms]} \qquad\qquad (4)$$

Ermittelt man für die 35 herzkreislaufgesunden liegenden Versuchspersonen diese arterielle Durchblutungsgüte aD, so erhält man als Normalwert bzw. -bereich:

$$N (aD) = 1 \pm 0{,}07.$$

Aus der Gleichung für aD folgt erfindungsgemäß in allgemeiner Form:

- *Je höher das Faser-Stretching und die Wendepunktzeit, desto geringer ist die arterielle Durchblutungsgüte.*
- Neben der arteriellen Durchblutungsgüte aD als Charakteristikum der peripheren Durchblutung ist der Zustand der elastischen Fasern, damit des Gefäßendothels, als Faser-Stretching FS ermittelbar. *Arterielle Durchblutungsgüte und Faser-Stretching sind umgekehrt proportional.*

Es wurde begründet, dass die Volumenpuls-Gipfelzeit $T_G$ ein Maß für die arterielle Durchblutung darstellt, gleichfalls die Wendepunktzeit $T_W$. Ermittelt man den statistischen Zusammenhang zur eingeführten arteriellen Durchblutungsgüte aD, so erhält man für die 120 Messungen bei den 29 Versuchspersonen die überzeugenden Resultate

- $r_{aD,\,TG} = - 0{,}93$,
- $r_{aD,\,TW} = - 0{,}88$.

Demnach ist es gleichfalls möglich, mittels Gipfelzeit eine *arterielle Durchblutungsgüte* (hier mit *aD\** bezeichnet) zu ermitteln, wenn auf den abgeleiteten Mittelwert $T_{G\;normal} = 213$ ms normiert wird:

$$aD^* = \frac{213}{T_G \, [ms]}$$

$$(5)$$

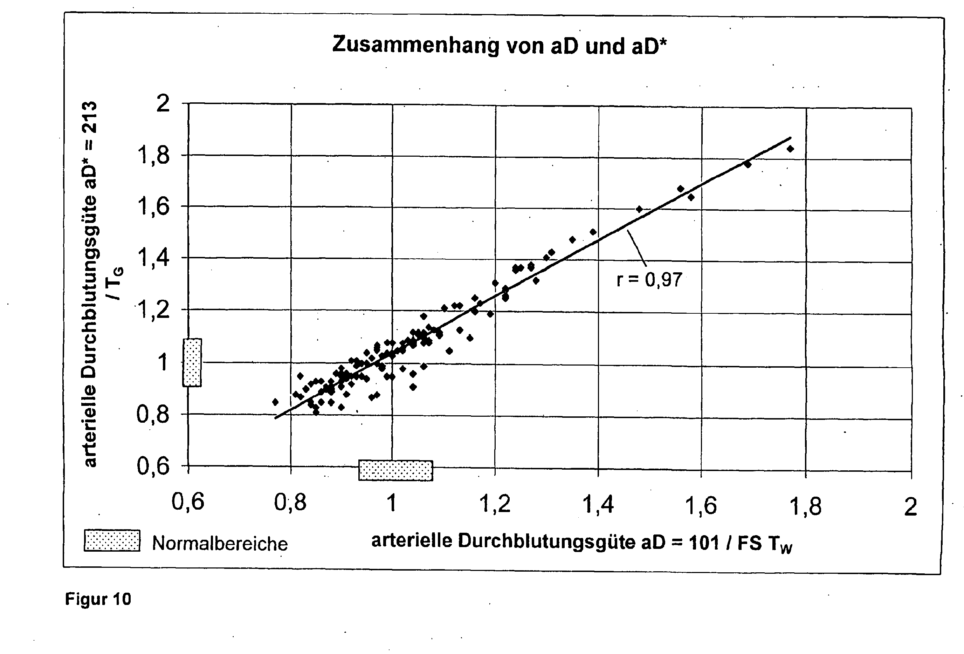

Bestimmt man die Korrelation von aD* mit aD, so ergibt sich für die 120 Messungen bei den 29 Versuchspersonen ein Wert von $r = 0,97$, die die These aD ≈ aD* bestätigt (Figur 10).

Ohne Kalibrierung ist bei nichtinvasiven Verfahren, wie es die NIRP-Methode darstellt, die Messung einer absoluten Mikrogefäß-Blutdurchflußmenge je Herzaktion nicht möglich. Aus den abgeleiteten Volumenpulskurven für das Blutvolumen (840nm) lassen sich jedoch *(relative) Blut-Füllungsvolumina* ableiten. Durch Ermittlung der Volumenpulsflächen (s. Figur 4) kann ein "*relatives mikrovaskuläres Blut-Füllungsvolumen*" als ein peripherer Kreislaufparameter bestimmt werden, da die Volumenpulsflächen den (relativen) Blutvolumenmengen entsprechen.

Zunächst werden die Pulskurvenminima durch eine jeweilige Gerade verbunden, wodurch die Pulsfläche basal eingegrenzt wird (Figur 4). Zwecks Normierung wird nun für eine entsprechende Messdauer (z. B. 64 s) die Fläche eines jeden Volumenpulses durch den berechneten mittleren Flächeninhalt dividiert. Der Mittelwert aller Beträge dieses relativen (normierten) mikrovaskulären Blut-Füllungsvolumens ist demnach für die zugrunde gelegte Messzeit (z. B. 64 s ) wegen der vorgenommenen Normierung immer gleich Eins und liefert eigentlich für die Bewertung keine Information, wohl aber die sich um diesen Mittelwert 1 einstellenden Blutvolumen-*Änderungen* bei den einzelnen Volumenpulsen n bzw. für jede Herzaktion. Für den *Normalfall* ist folgender Verlauf der Füllungsvolumina charakteristisch:

- Es bildet sich bei diesen relativen peripheren mikrovaskulären Blutfüllungsvolumina die *Atmungsperiodik* ab, ausgedrückt durch ein Vielfaches einer Herzaktion (Pulsnummer n).
- Als Maß für die Abweichung vom Mittelwert 1 wird die Standardabweichung (Wurzel aus dem mittleren quadratischen Wert), die Variabilität des Blutfüllungsvolumens, bestimmt.

Nichtnormalfälle (z. B. Diabetes mellitus bzw. koronare Erkrankungen) weichen davon z. T. extrem ab.

Erfindungsgemäß werden neben den Füllungsvolumina für das Blutvolumensignal (840nm-Signal) sowie das oxymetrische Signal (640nm-Signal) entsprechend bisheriger Arbeiten analoge Volumina für die NO-Wellenlänge von abkürzend ca. 400 nm abgeleitet und zueinander in Relationen gesetzt: 400 zu 840 nm, 400 zu 640 nm. So sind bei normaler Oxygenierung die Füllungsvolumina bei 840 und 640 nm nahezu deckungsgleich, während sich wesentliche Unterschiede z. B. bei auftretenden Vasokonstriktionen einstellen. Analoges gilt unter Einbeziehung der NO-Wellenlänge.

Sollen die Füllungsvolumina bei angelegtem Sensor unterschiedlicher Messungen, also zu verschiedenen Zeitpunkten (z. B. vor, während und nach einer Therapie; bei speziellen Funktionstests, wie Ruhe- und nachfolgender Hyperventilation), verglichen werden, so kann durch Division der Volumenpulsflächen der aktuellen Messung und dem Pulsflächen-Mittelwert der sogenannten "Bezugsmessung" die auftretende Veränderung sowohl vom mikrovaskulären Blutvolumen als auch vom oxymetrischen Signal bzw. zum NO-Pulsationssignal quantitativ dargestellt werden.

Im Sinne der Geometrie wird zwischen dem 840 nm - und dem 640 nm - Blutvolumensignal eine "Ähnlichkeit" bestimmt, so dass hierfür als quantitative Größe und damit als "*Form-Ähnlichkeitsmaß*" dieser Signale der bekannte *Korrelationsfaktor r* ermittelbar ist. Dieser Korrelationsfaktor wird in [Europäische Patentanmeldung Nr. 97118971.7: Vorrichtung und Arbeitsverfahren zum Betreiben der Vorrichtung zur nichtinvasiven Messung und Auswertung von Kreislauf-Kennfunktionen und Parametern] *peripherer Mikrozirkulations-Koeffizient pMC* genannt: $r = pMC$. Dabei wird pMC je Herzaktion n ermittelt, ebenso werden daraus für eine bestimmte Messzeit ein arithmetischer Mittelwert sowie die Standardabweichung abgeleitet. Damit ist eine quantitative Beurteilung des Zusammenwirkens von Blutfluß, Gefäßen sowie vasomotorischer Fasern peripherer Nerven möglich. Es stellt sich z. B. bei Hyperämisierung am Meßort der periphere Mikrozirkulations-Koeffizient pMC ≈ 1 ein, während im Falle einer Vasokonstriktion ein z. T. beträchtlich reduzierter Wert erhalten wird, wie auch die medizinischen Evaluierungen zeigten [u. a. Krauß, M, G. Grohmann, J. Addae, Ch. Posthoff et R. Saunders: Non-invasive Recording of Parameters of the Heart and the Circulatory System Using the NIRP-Method and its Application for the Sickle Cell Anaemia (liegt als noch nicht veröffentlichtes Manuskript vor). Erfindungsgemäß wird nun dieses Form-Ähnlichkeitsmaß pMC verallgemeinert: Bestimmung der Ähnlichkeit zwischen den Pulsationen der NO-Wellenlänge bei ca. 400 nm sowie bei 840 nm (mikrovaskuläres Blutvolumen) bzw. zwischen den Pulsationen der NO-Wellenlänge und denen bei 640 nm (oxymetrisches Signal). Dadurch lassen sich in Echtzeit NO-Veränderungen ermitteln, die mit bisherigen Methoden zur direkten Messung des freien NO-Gases (u. a. Gasphasen-Chemilumineszenz, Elektronenspinresonanz (ESR), Nitratbestimmung) nicht möglich sind bzw. sich als zu aufwendig und in der Quantifizierung als unsicher erwiesen, da die NO-Halbwertzeit sehr kurz ist (nach [Schmidt, H.H.H.W.: NO, endogener Botenstoff und Zellgift. MED. MO. PHARM. 17, 6 (1994), 168 - 185] ca. 5 s) und NO eine hohe Reaktivität mit Sauerstoff und anderen Zellbestandteilen besitzt. Als Formähnlichkeitsmaße werden je Pulsation n und daraus als Mittelwert einschließlich Standardabweichung erfindungsgemäß bestimmt:

- zwischen den Wellenlängen 840 und ca. 400 nm:

  peripherer Stickstoffmonoxid(NO)-induzierter Koeffizient $pNOC_1$

- zwischen den Wellenlängen 640 und ca. 400 nm:

  peripherer Stickstoffmonoxid(NO)-induzierter Koeffizient $pNOC_2$.

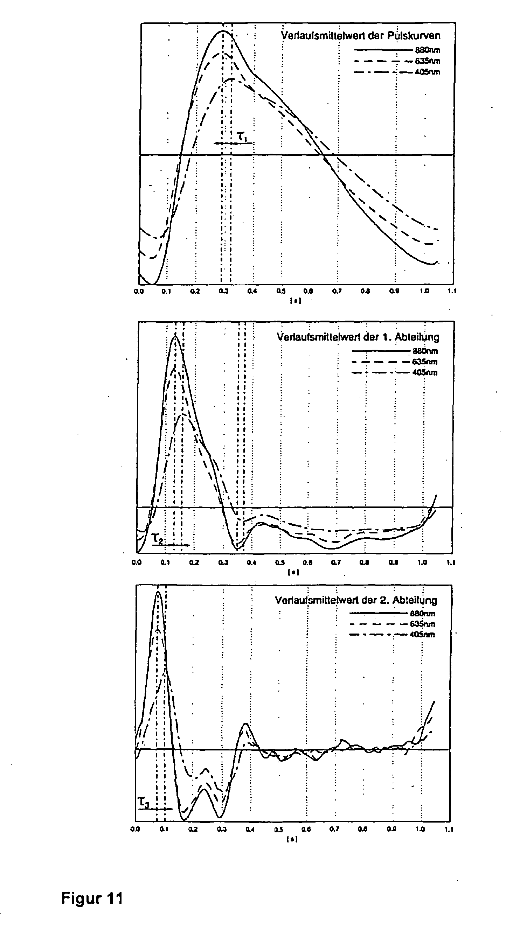

Leitet man von einem peripheren Messort mittels Drei-Wellenlängen-NIR-ROT-NO-Remissions-Photoplethysmographie und den Wellenlängen 880, 635 und 405 nm die sich einstellenden Pulsationen ab, so erhält man, wie aus Figur 11 bei einer 28jährigen liegenden Normalperson (Messort Fingerbeere) hervorgeht, Unterschiede bei den Formen einer Pulsation (Formähnlichkeitsmaß ≠ 1). Wie erkannt wurde, lassen sich spezielle Zeitmaße als Parameter zwischen den Pulsationen der einzelnen Wellenlängen als Charakteristika wie folgt bestimmen:

- Während zwischen den Pulskurven bei 880 und 635 nm nahezu Deckungsgleichheit auftritt, erhält man im vorliegenden Fall zwischen 880 und 405 nm einen (positiven) Zeitversatz

$$\tau_1 = T_{G\ 405\ nm} - T_{G\ 880\ nm} > 0 \tag{6}$$

beim Erreichen des Gipfels, also bei den Gipfelzeiten $T_G$: Nachlaufen des Gipfels bei 405 nm.
- Analoges Verhalten stellt sich bei den 1. Ableitungen ein (Blutvolumengeschwindigkeit): Während die Wendepunktzeiten bei 880 und 635 nm nahezu übereinstimmen, ist die Wendepunktzeit bei 405 nm erhöht, so dass für diesen Zeitversatz gilt

$$\tau_2 = T_{w\ 405\ nm} - T_{w\ 880\ nm} > 0 \tag{7}$$

- Gleiches gilt für die 2. Ableitung (Blutvolumenbeschleunigung, Figur 4):

$$\tau_3 = T_{BE\ 405\ nm} - T_{BE\ 880\ nm} > 0. \tag{8}$$

Der wichtigste endogene Vermittler einer *Vasodilatation,* Stickstoffmonoxid (NO), spielt bei der gesamten endothelvermittelten Durchblutungsregulation eine dominante Rolle [Scriba, P. C. und S. Endres: NO. Mittler, Missetäter und Medikament. Sonderheft Internist 1997. 38:405 © Springer 1997; Schmidt, H.H.H.W.: NO, endogener Botenstoff und Zellgift. MED. MO. PHARM. 17, 6 (1994), 168 - 185; Kelm, M.: Kardiovaskuläre Wirkungen von Stickstoffmonoxid und ihre Bedeutung für die arterielle Hypertonie. Schattauer Stuttgart/New York 1996]. Unter dem Einfluß verschiedener Faktoren kommt es zu einer gesteigerten NO-Freisetzung [Thews, G. und P. Vaupel: Vegetative Physiologie. 3. Aufl. Springer Berlin/Heidelberg/New York/Barcelona/Budapest/ Hongkong/ London/ Mailand/Paris/Santa Clara/Singapur/ Tokio 1997]. Derartige fördernde Faktoren, die zu einer Gefäßdilatation führen, sind u. a.:

- eine erhöhte Schubspannung des strömenden Blutes sowie die durch das strömende Blut auf die Glykokalyx des Endothels ausgeübte tangentiale Kraft, die bei Erhöhung der Strömungsgeschwindigkeit überproportional zunimmt und damit die NO-Freisetzung verstärkt,
- die durch die Herzaktionen erzeugten arteriellen Gefäßpulsationen selbst, die mit der NIRP- Methode erfasst werden.

Die Veränderung des Gefäßendothels bis hin zu einer endothelialen Dysfunktion ist eng mit laminarer und turbulenter Strömung des Blutes verbunden. Von Ritz Ritz E: Exempla hypertonica: Bildatlas zur Pathophysiologie und Pharmakologie der Hypertonie. München: Med. Service, Bd. 2, 1990] sowie *Schmid-Schönbein* [Schmid-Schönbein, H; Grunau, G und H Bräuer: Exempla hämorheologica, Albert-Roussel Pharma GmbH, Wiesbaden, 1980] wird dargestellt, dass die einfache laminare Strömung, wie sie offensichtlich bei dem Normalprobanden in Figur 5a gegeben ist, auf einer Verschiebung von Flüssigkeitsschichten gegeneinander beruht. Der dabei auftretende Widerstand beruht nach *Schmid-Schönbein et al* letztlich auf der inneren Reibung, also der Viskosität der Flüssigkeit. Anders liegen die Verhältnisse bei turbolenter Strömung, wie sie bei Auftreten einer Mediasklerose nach Figur 5c vorliegen können. Grundsätzlich kommt es bei pulsatorischer Fluktuation des Drucks zur starken Durchmischung der Grenzschicht. Zonen hoher Geschwindigkeit gelangen dadurch auch in unmittelbare Nähe der Gefäßwand, so dass z. T. extreme Schubspannungen entstehen, wie sie vor allem bei Hochleistungssportlern auftreten. Dies führt zur anormalen Freisetzung von NO und damit zur massiven Vasodilatation. *Ritz* verweist darauf, dass durch den dabei entstehenden Reibungswiderstand der turbolenten Grenzschicht es zur Verletzung des für das arterielle Gefäßsystem so wichtigen Endothels kommen kann, was ebenfalls zu einer Endotheldysfunktion führt.

Es ist demnach durch gezielte Veränderungen vor allem der Schubspannungen und damit durch spezielle externe Verfahren bzw. Stimuli möglich, gezielt Einfluß auf eine Freisetzung von NO zu nehmen. [WO02/34105 A2: Method an Apparatus for non-invasively evaluating endothelial activity in a patient].

Eine sich einstellende Blutgefäßdilatation durch NO-Aktivierung muß stets zu einer Verringerung des Gefäßtonus (s. Figur 3a) führen. Darüber hinaus reagiert das Radikal NO in biologischen Flüssigkeiten innerhalb einer sehr kurzen Zeit mit $O_2$ und Wasser zu einem Gemisch aus Nitrit und Nitrat [Kelm, M.: Kardiovaskuläre Wirkungen von Stickstoffmonoxid und ihre Bedeutung für die arterielle Hypertonie. Schattauer Stuttgart/New York 1996], so dass sich am Messort als Ausdruck der erfolgten NO-Oxidation eine Reduzierung der pulsatilen arteriellen $O_2$-Sättigung [Europäische Patentanmeldung Nr. 97118971.7: Vorrichtung und Arbeitsverfahren zum Betreiben der Vorrichtung zur nichtinvasiven Messung und Auswertung von Kreislauf-Kennfunktionen und Parametern] einstellen muß, falls eine erhöhte Schubspannung des strömenden Blutes die Ursache war. Eine mit der nichtinvasiven NIRP-Methode abgeleitete Gefäßdilatation, verursacht durch eine NO-Aktivierung, muß sich demnach allgemein wie folgt darstellen:

- Zunahme der peripheren Durchblutung
- Rückgang des Gefäßtonus
- Abnahme der pulsatilen arteriellen $O_2$-Sättigung.

Reduziert sich jedoch die periphere Durchblutung, erhöht sich der Gefäßtonus sowie die pulsatile arterielle $O_2$-Sättigung, dann kann offenbar nicht von einer NO-Erhöhung ausgegangen werden. Vielmehr erhöht sich der Anteil der gefäßverengenden Substanzen, das Systemgleichgewicht im Endothel hat sich verändert, eine endotheliale Dysfunktion ist wahrscheinlich.

In diesem Sinne soll methodenkritisch festgestellt werden, dass es bei dem Verfahren zur Ermittlung einer endothelialen Dysfunktion nach *Celermajer* [Celermajer DS, Sorensen KE, Gooch VM, Spiegelhalter DJ, Miller OI, Sullivan ID, Lloyd JK, Deanfield JE: Non-invasive detection of endothelial dysfunction in children and adults at risk of atherosclerosis. Lancet. 1992;340(8828):1111-5, Celermajer DS: Testing endothelial function using ultrasound. J Cardiovasc Pharmacol. 1998;32 Suppl 3:S29-32] trotz Gabe eines Vasodilatators und nachfolgender Wiederholung der Messprozedur für den Durchmesser der Arteria brachialis (Oberarmarterie) nicht möglich ist, eine direkte Aussage über eine erfolgte NO-Oxidation zu treffen, da weder die Veränderung der Sauerstoff-Sättigung noch die von NO erfasst wird, was aber für die Bestimmung einer endothelialen Dysfunktion als Endothel-Fehlverhalten bezüglich der NO-Freisetzung unbedingt erforderlich ist. Es wird mit dieser Methode lediglich eine Gefäßdilatation unter Gabe eines Vasodilators festgestellt, um die Elastizität der Gefäßwand unter Ausschaltung des Endothels zu beurteilen. Bei der Gefäßdilatation nach Öffnen der arteriellen Sperre entsteht eine Mehrdurchblutung (reaktive Hyperämie), die durch NO bedingt ist, so dass indirekt auf den Funktionszustand des Endothels geschlossen werden kann. Ebenso werden nur die nach dem Test sich einstellenden stationären Verhältnisse bewertet, keine Übergangsprozesse.

Im Sinne der (linearen) Systemtheorie wird bekanntlich eine Systemstabilität derart bestimmt, dass eine *definierte, zeitlich veränderliche Eingangsgröße* (z. B. Sprung- oder Stoßfunktion) am Systemeingang angelegt wird. Analysiert wird die sich einstellende *zeitlich veränderliche Systemausgangsgröße.* Verläuft sie "instabil", also bis zu einer gewissen Aussteuerungsgrenze, dann liegt eine Systeminstabilität vor [Krauß, M. und E.-G. Woschni: Meßinformationssysteme. Kennfunktionen, Gütekriterien, Optimierung. 2.Aufl. VEB Verlag Technik 1975].

Ein solcher Systemtest als *Funktionstest zur Bestimmung einer endothelialen Dysfunktion* wird wie bereits aus der Offenlegungsschrift WO 02/34105 A2 [Method an apparatus for non-invasively evaluating endothelial activity in a patient] bekannt ist, wie folgt realisiert:

- Anlegen einer Blutdruck-Staumanschette am Ober- bzw. Unterarm oder Ober- bzw. Unterschenkel einer liegenden Person,
- Ableitung der mikrovaskulären Blutvolumenpulsationen, der zugehörigen oxymetrischen sowie der "NO-Pulsationen" (Wellenlänge ca. 400 nm) vom entsprechenden peripheren Messort (Finger- bzw. Großzehenbeere) der liegenden Person, nachdem sich ein vaskuläres Gleichgewicht eingestellt hat (nach ca. 2 bis 3 Minuten Liegezeit erreicht). NIRP-Messzeit etwa 2 Minuten,
- mittels Staumanschette Erzeugung einer Kompression, bis die Pulsationen verschwinden (Druck z. T. bis etwa 250 mmHg ),

- Stauzeit 5 ... 10 Minuten,
- sprungartiges Öffnen der Manschette, Erfassung und Auswertung der peripheren Pulsationen (u. a. Veränderungen der mikrovaskulären Blutfüllungsvolumina, der arteriellen Durchblutungsgüte, des Faser-Stretching, der pulsatilen Sauerstoff-Sättigung, der NO-Blutfüllungsvolumina) als sich einstellende Systemübergangsfunktionen. Messzeit bis etwa 5 Minuten.

Durch das sprungartige Öffnen der angelegten Blutdruckmanschette erhöht sich stark die Schubspannung des strömenden Blutes, im Normalfall verstärkt sich in

Form einer zeitlichen Übergangsfunktion die Freisetzung des Vasodilatators NO im Vergleich zum Zeitpunkt vor der Stauung. Ein derartiger Funktionstest muß neben einer Vasodilatation zu einer Reduzierung der pulsatilen $O_2$-Sättigung im Normalfall führen, im Zeitpunkt unmittelbar nach Öffnung muß das Faser-Stretching erhöht und die arterielle Durchblutungsgüte entsprechend reduziert sein. *Durch Anlegen einer Kompression und sprungartiges Öffnen der Staumanschette ergeben sich im Normalfall zeitlich veränderliche Übergangsfunktionen für entsprechende hämodynamische Parameter.*

Die Erfindung soll anhand eines Ausführungsbeispiels und von Figuren näher erläutert werden.

Hierbei zeigen:

**Figur 1:** Speicherungs- und Entspeicherungsfunktion ("Windkesselfunktion") im arteriellen Gefäßsystem [nach Thews, G. und P. Vaupel: Vegetative Physiologie. 3. Aufl. Springer Berlin/Heidelberg/New York/Barcelona/Budapest/ Hongkong/ London/ Mailand/Paris/Santa Clara/Singapur/Tokio 1997]. In der Aorta ascendens bis zur arteriellen Gefäßperipherie führt der Auswurf des Schlagvolumens zu einer Wanddehnung mit anschließender Entdehnung.

**Figur 2:** Prinzip der nichtinvasiven NIRP-Methode: Wird im Wellenlängen-Multiplexbetrieb gepulstes Licht des Nahen-Infrarot-Bereiches der Wellenlänge S1 = 840 nm in das Gewebe eingeleitet, so werden relativ große Gewebevolumina bei Eindringtiefen von ca. 3...4 mm illuminiert. Die entstehenden Rückstreusignale werden über einen Empfänger E erfaßt und diagnostisch bewertet. Speziell lassen sich mit 840nm [Nahes Infrarot, Hb/HbO$_2$-remissions-isosbestscher Bereich] die das Mikrogefäßsystem am speziellen Meßort je Zeiteinheit passierenden (relativen) Mengen des Blutes bzw. der Erythrozyten, unabhängig vom Oxygenierungsgrad, ableiten. Dagegen wird mit der Wellenlänge S2 = 640 nm der Oxygenierungsgrad dieser durch 840 nm erfaßten Blut- bzw. Erythrozytenmenge ermittelbar. Aus den im Empfänger E sich jeweils einstellenden Rückstreusignalen lassen sich Änderungen sowohl der mikro- als auch makrovaskulären Durchblutung einschließlich der Oxygenierung ableiten, jedoch mit ausreichender Genauigkeit auch auf kardiovaskuläre Parameter schließen, da die Pulsperioden mit denen der zugehörigen Herzaktionen übereinstimmen. Des weiteren lassen sich mit weiteren Wellenlängen zusätzliche Parameter erfassen (z. B. kann mit S3 $\approx$ 400 nm auf Stickstoffmonoxid als Gefäßdilator geschlossen werden). Zur Kontrolle der Temperatur δ am Meßort ist ein entsprechender Sensor im Clip integriert.

**Figur 3:** a) Mikrovaskuläre Blutvolumenpulsation am Meßort Fingerbeere bei einem 25jährigen liegenden Normalprobanden unter Ruheventilation, abgeleitet mit der nichtinvasiven NIRP-Methode, b) zugehörige Blutvolumenpulsationen in einem 4-Sekunden-Zeitfenster einschließlich charakteristischer Zeitparameter.

**Figur 4:** Mikrovaskuläre Blutvolumenpulsation und Definition des Blutfüllungsvolumens. Aus der Blutvolumenpulsation abgeleitete Blutvolumengeschwindigkeit einschließlich dort markante Flächen F$_1$ und F$_2$ sowie Zeitabschnitte, um daraus das Dehnungsmaß "Faser-Stretching FS" sowie die arterielle Durchblutungsgüte aD zu bestimmen. Die Blutvolumenbeschleunigung als 2. Ableitung der mikrovaskulären Blutvolumenpulsation.

**Figur 5:** Mikrovaskuläre Blutvolumenpulsationen sowie daraus abgeleitete Blutvolumengeschwindigkeiten einschließlich markante Zeitabschnitte bei charakteristischen Fällen am jeweiligen Messort Großzehenbeere (Figur 5a 22jähriger Normalproband, Figur 5b 63jähriger TypII-Diabetiker mit peripherer arterieller Verschlusskrankheit sowie Figur 5c 68jähriger TypII-Diabetiker mit Mediasklerose). Deutlich unterscheidet sich die Form der Blutvolumengeschwindigkeiten innerhalb einer Pulsperiode, so dass daraus sowohl qualitative als auch quantitative Maße ableitbar sind.

**Figur 6:** Abhängigkeit des relativen peripheren Widerstandes RPW$_2$ vom Faser-Stretching FS an Finger- und Großzehenbeeren bei 29 Versuchspersonen mit ausgebildeten Dikrotien [15 männl., 14 weibl., mittleres Alter 23,5 J.]. Dabei handelt es sich um 11 Herzkreislaufgesunde, 9 Leistungssportler (ca. 12 Stunden nach Wettkampf), 7 Sichelzellpatienten, 2 Typ I-Diabetiker.

**Figur 7:** Zusammenhang zwischen dem Zeitquotienten $T_B/T_W = (T_G-T_W)/T_W$ und dem Faser-Stretching FS an Finger- und Großzehenbeeren bei den 29 Versuchspersonen nach Figur 6.

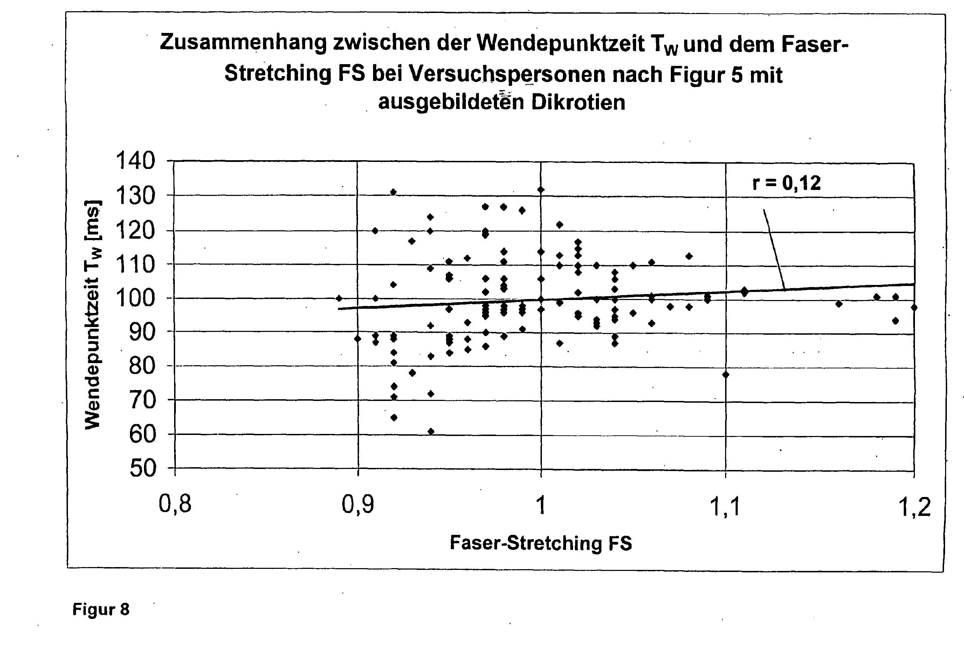

**Figur 8:** Abhängigkeit zwischen der Wendepunktzeit $T_W$ und dem Faser-Stretching FS bei Versuchspersonen nach Figur 6, d.h. bei Personen mit relativ stark ausgeprägten Dikrotien.

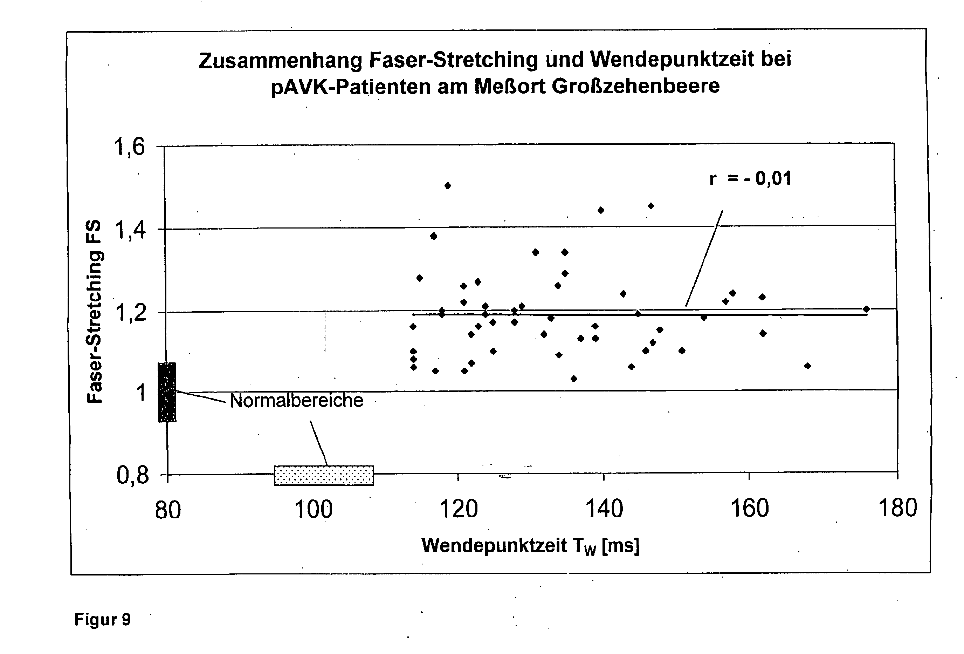

**Figur 9:** Abhängigkeit zwischen der Wendepunktzeit $T_W$ und dem Faser-Stretching FS bei 50 pAVK-Patienten, abgeleitet von den Messorten Großzehenbeere.

**Figur 10:** Vergleich der ermittelten arteriellen Durchblutungsgüten aD und aD*, abgeleitet aus den 120 Messungen der 29 Versuchspersonen nach Figur 6.

**Figur 11**: Von dem peripheren Messort Fingerbeere bei einer 28jährigen liegenden Normalperson mittels Drei-Wellenlängen-NIR-ROT-NO-Remissions-Photoplethysmographie und den Wellenlängen 880, 635 und 405 nm abgeleitete Pulsationen einschließlich 1. und 2. Ableitung sowie auftretende Zeitmaße (Zeitversätze) $\tau_1$, $\tau_2$ $\tau_3$.

**Figur 12:** Arterielle Durchblutungsgüte aD und Faser-Stretching FS, abgeleitet vom NIRP-Meßort Großzehenbeere, bei 50 Patienten mit peripherer arterieller Verschlusskrankheit.

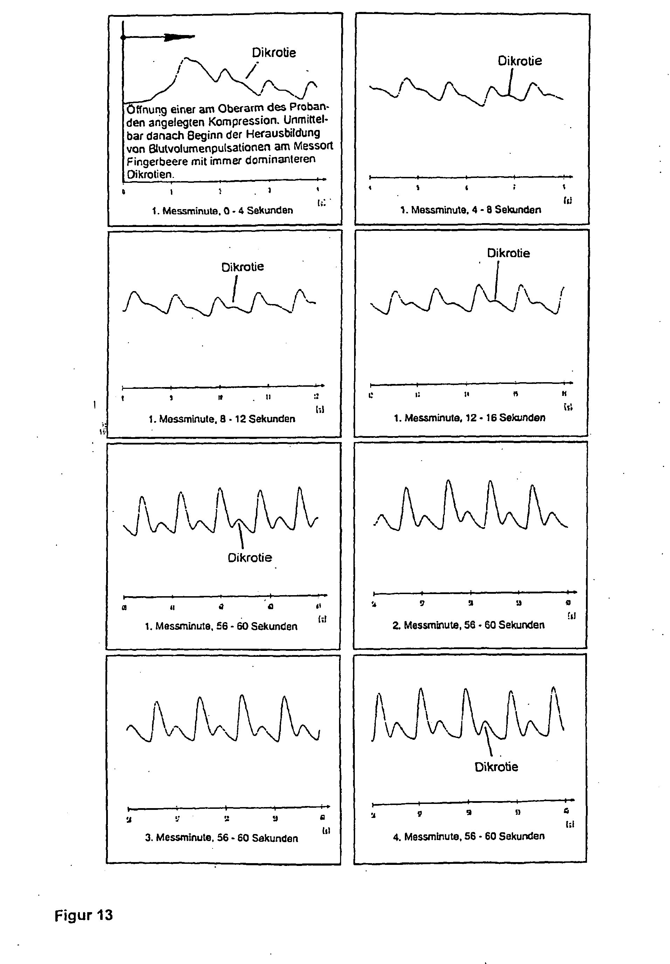

**Figur 13**: Form der Blutvolumenpulsationen, abgeleitet vom Messort 2. Fingerbeere re. bei einem 27jährigen Probanden (Radsportprofi), wenn eine am Oberarm angelegte Kompression von 250 mm Hg plötzlich geöffnet wird.

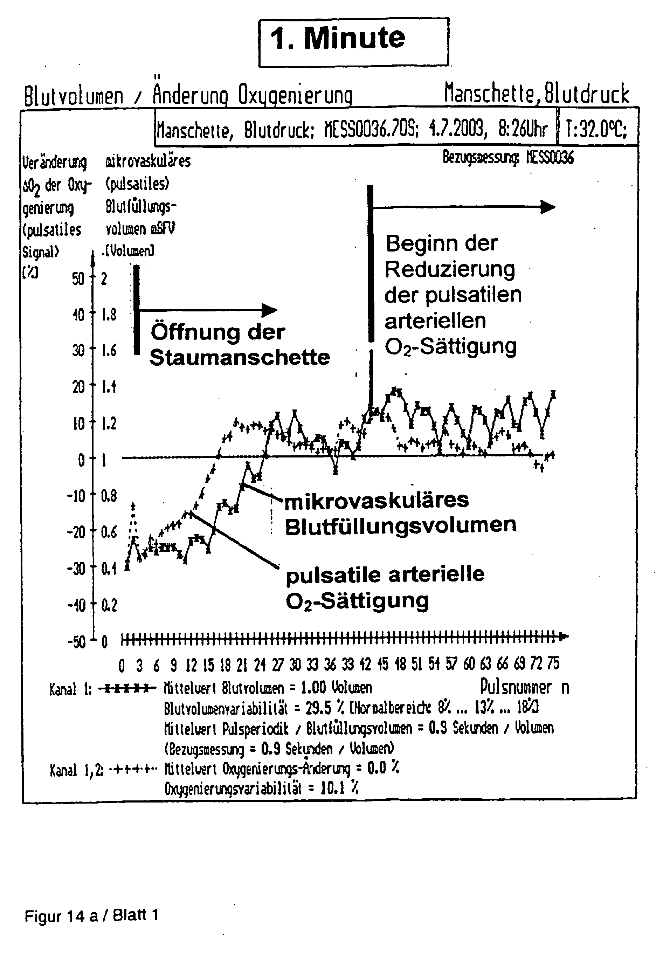

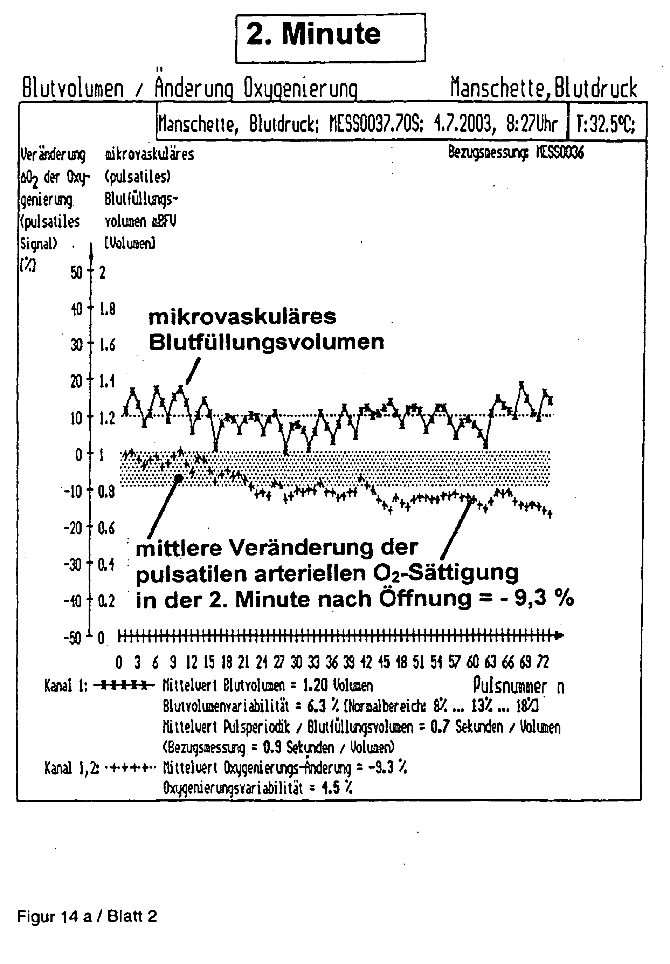

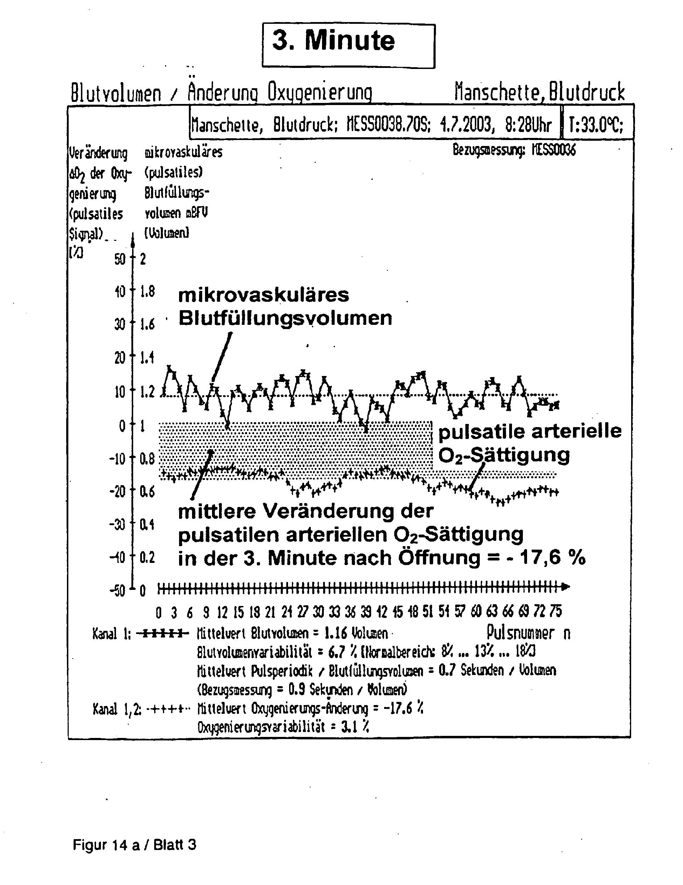

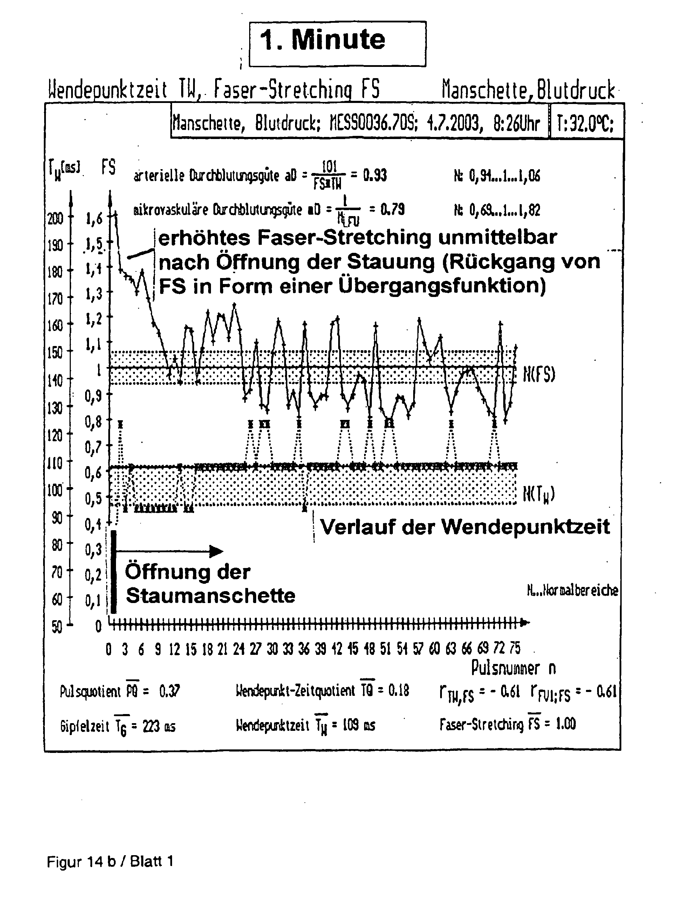

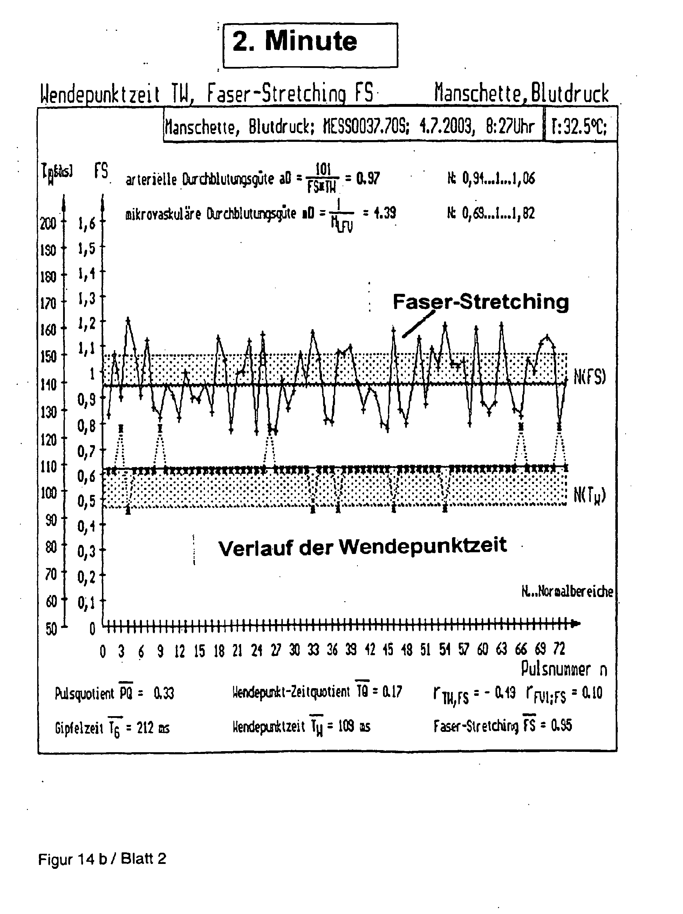

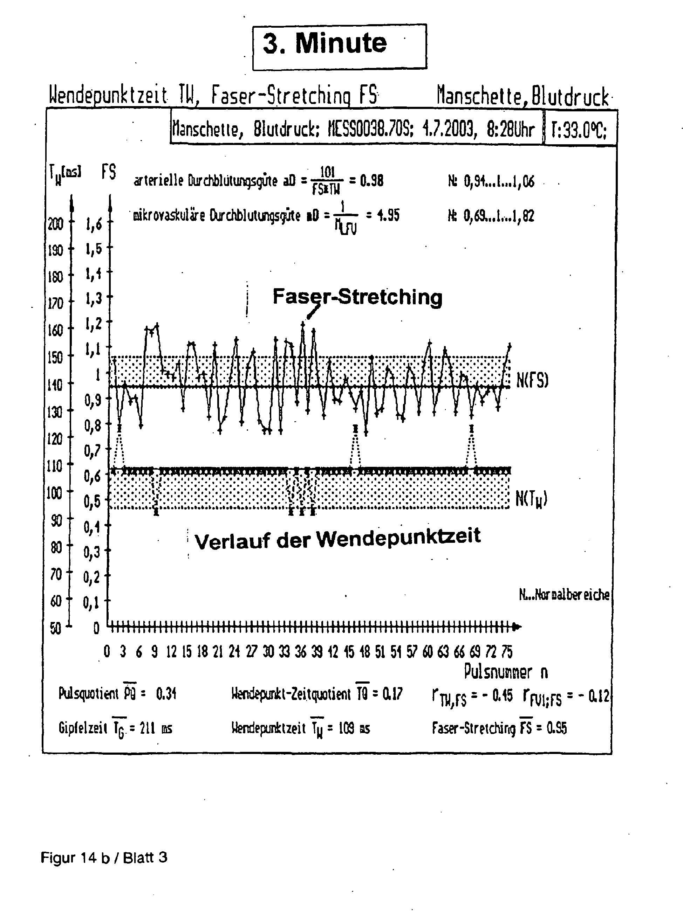

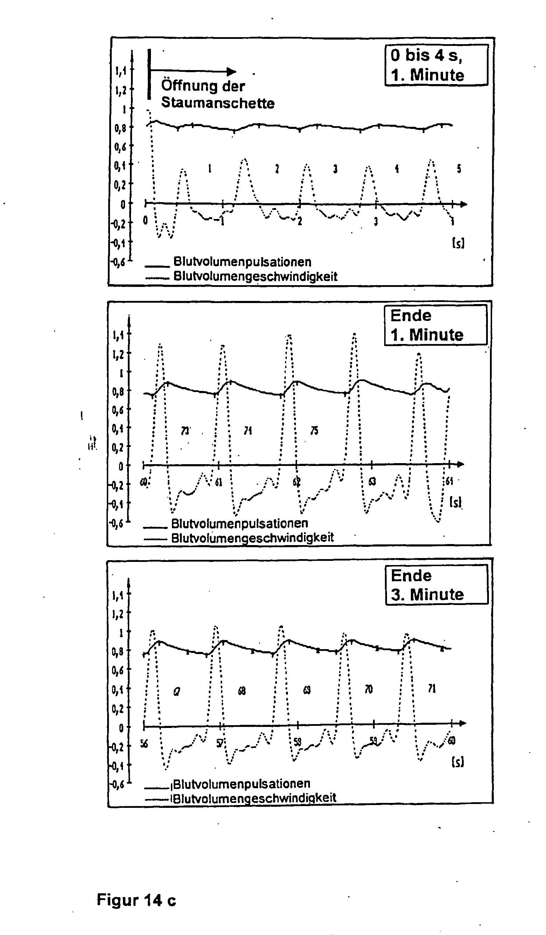

**Figur 14:** Verläufe des mikrovaskulären Blutfüllungsvolumens und der pulsatilen arteriellen $O_2$-Sättigung (Figur 14a), von Faser-Stretching und der Wendepunktzeit (Figur 14b) sowie von Form und Amplitude von Blutvolumenfluß und daraus abgeleiteter Blutvolumengeschwindigkeit (Figur 14c) bei einem 55jährigen Normalprobanden am Messort Fingerbeere, wenn eine am Oberarm angelegte Kompression von 250 mm Hg plötzlich geöffnet wird.

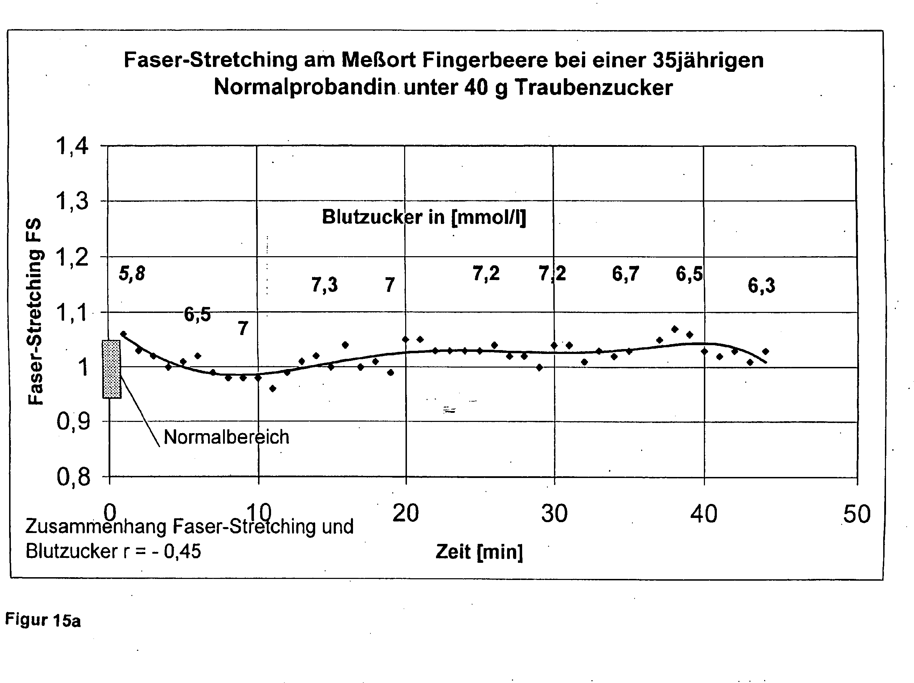

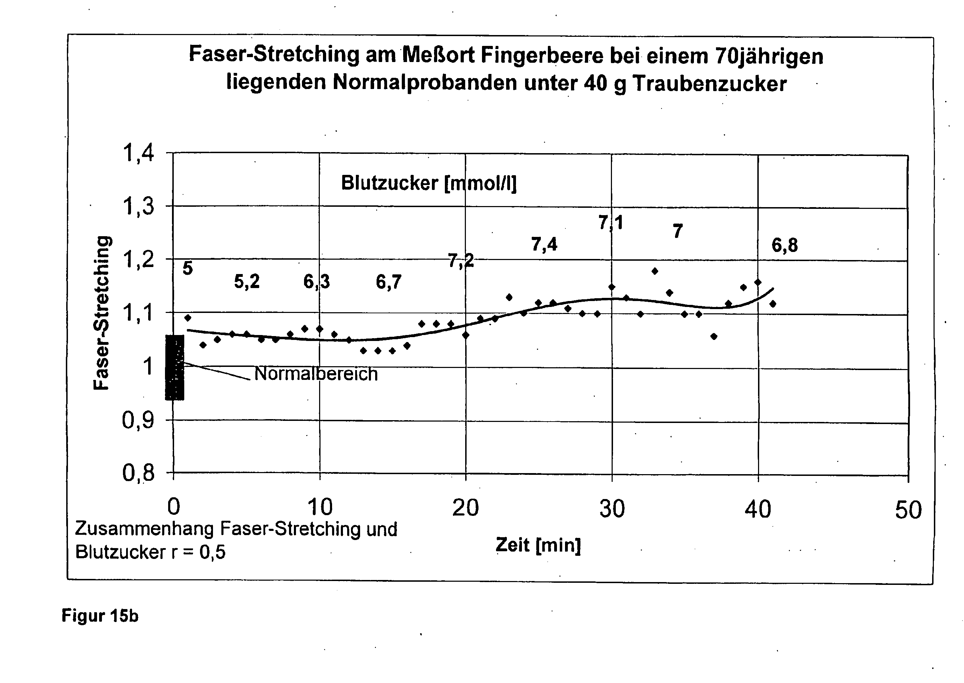

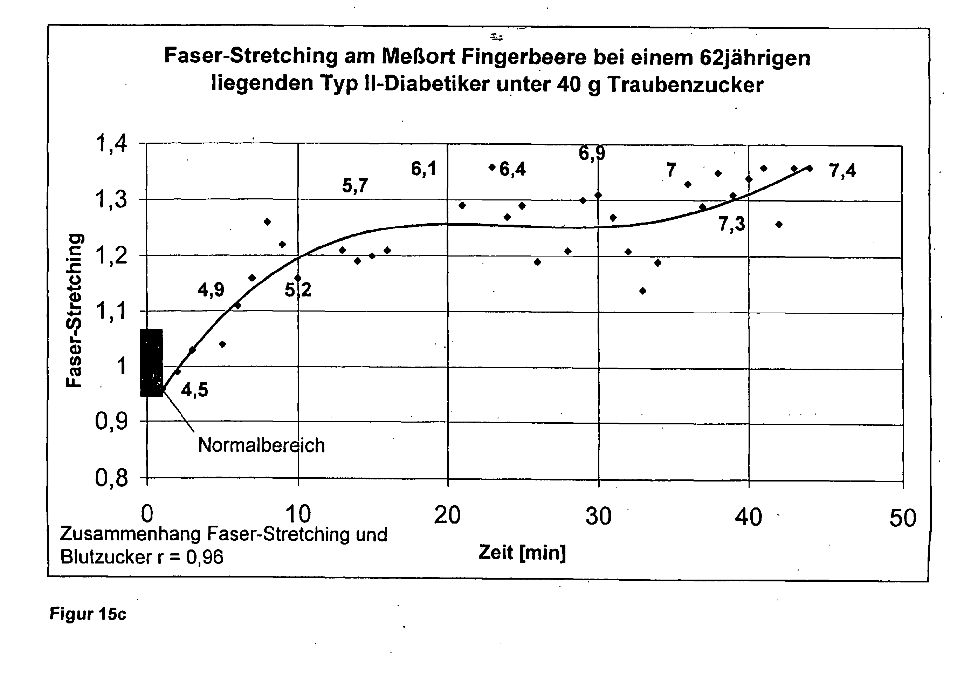

**Figur 15:** Charakteristische Verläufe des peripheren Faser-Stretching bei Normalpersonen (Figur 15a, b) und einem Typ II-Diabetiker (Figur 15c), die unter jeweiliger Gabe von 40 g Traubenzucker als Funktions- bzw. Stimulationstest vom Messort Fingerbeere abgeleitet wurden.

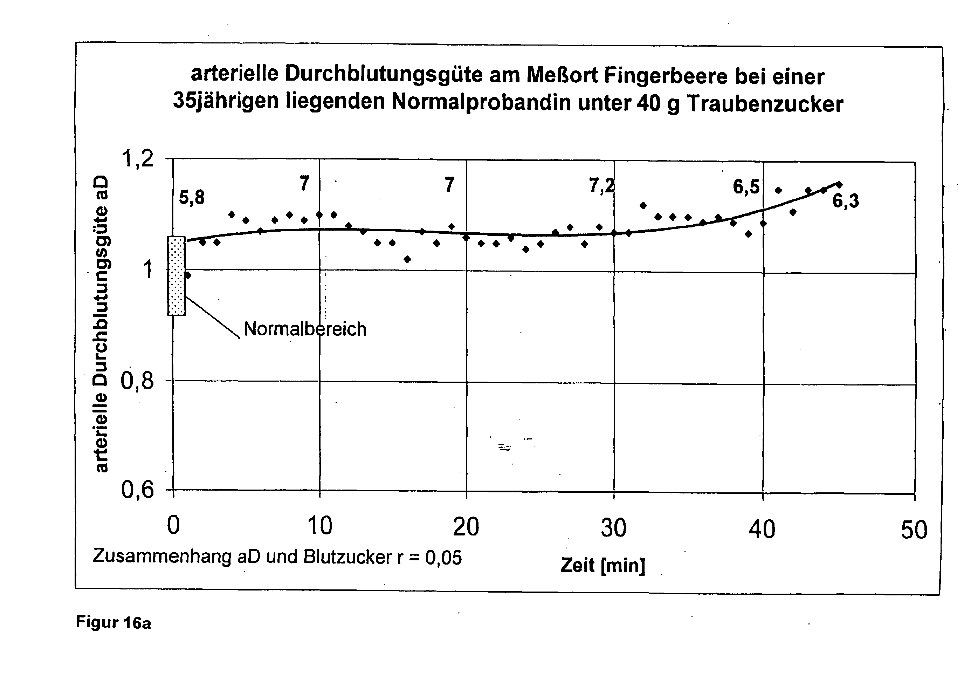

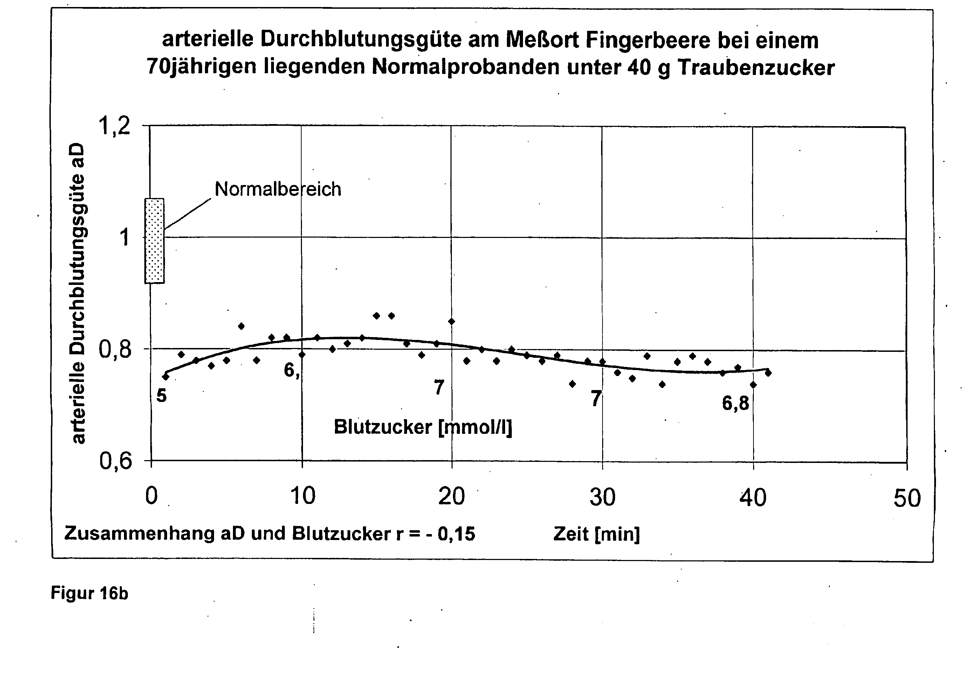

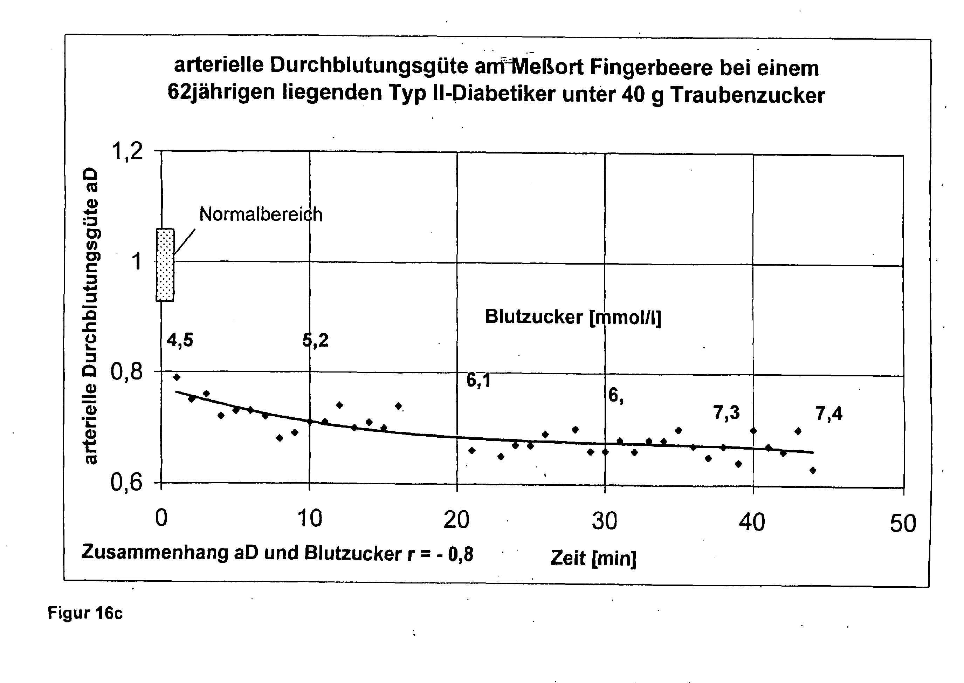

**Figur 16:** Veränderungen der arteriellen Durchblutungsgüte aD am Messort Fingerbeere, die sich unter Gabe von 40 g Traubenzucker bei den Versuchspersonen nach Figur 15 einstellten.

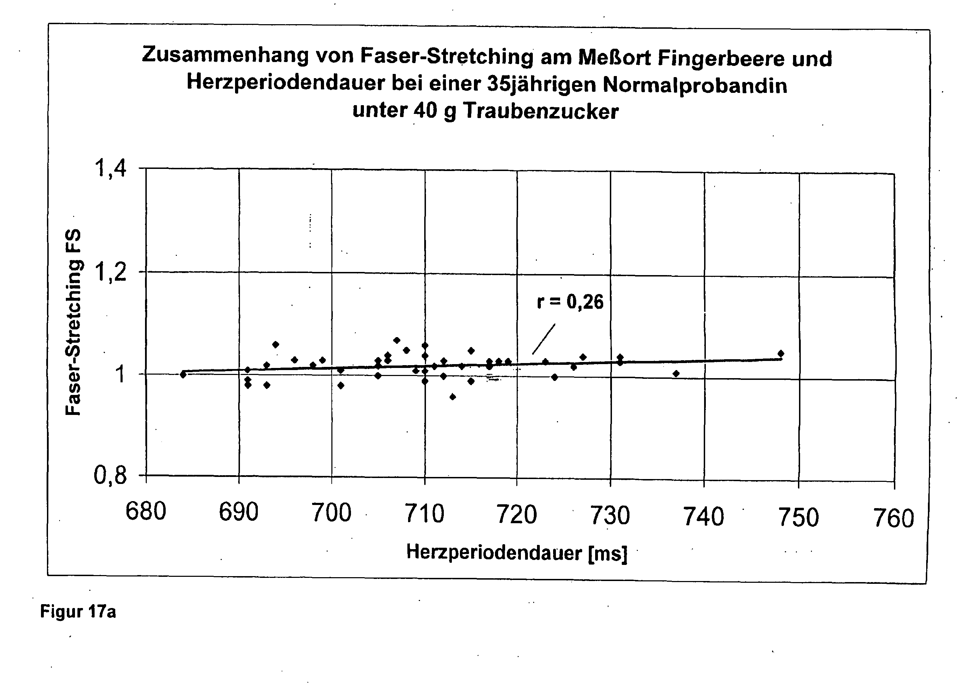

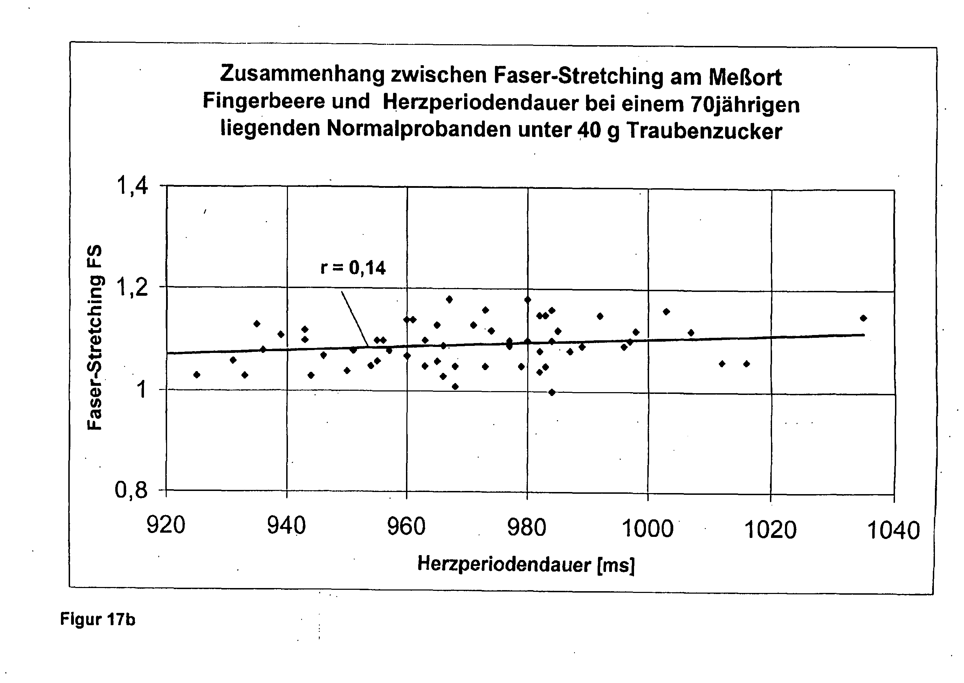

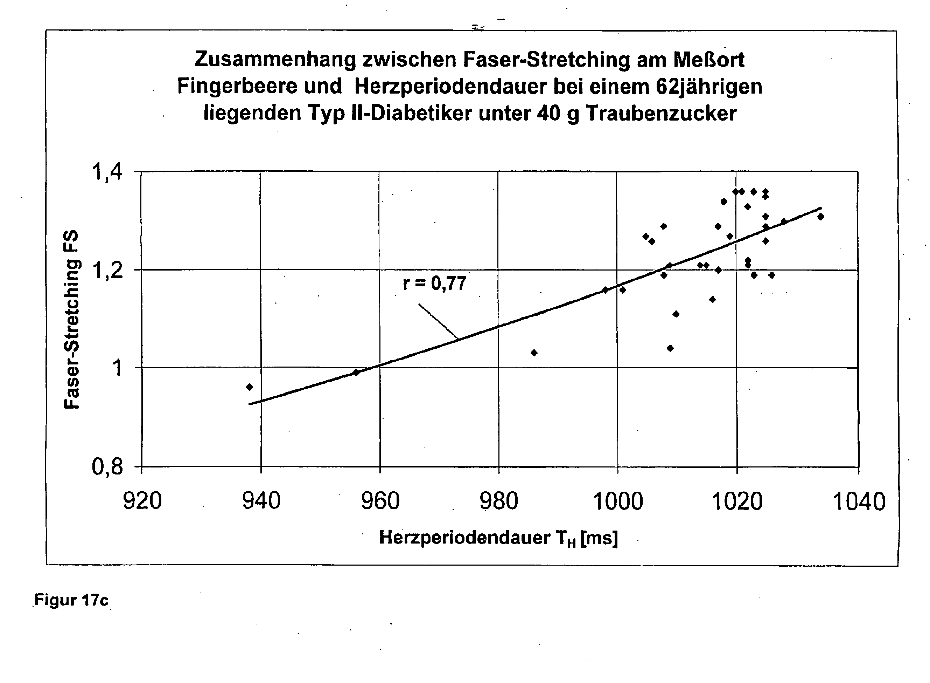

**Figur 17:** Zusammenhang zwischen Faser-Stretching, abgeleitet vom peripheren Meßort Fingerbeere, sowie der Herzperiodendauer bei Normalpersonen und Typ II-Diabetikern nach den Figuren 15 und 16 unter Gabe von 40 g Traubenzucker.

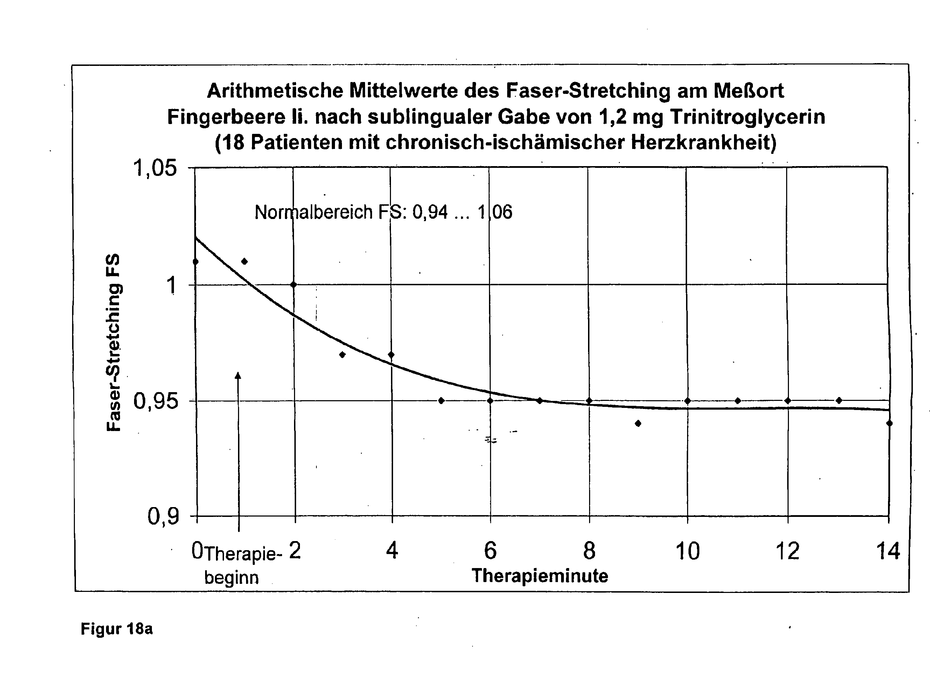

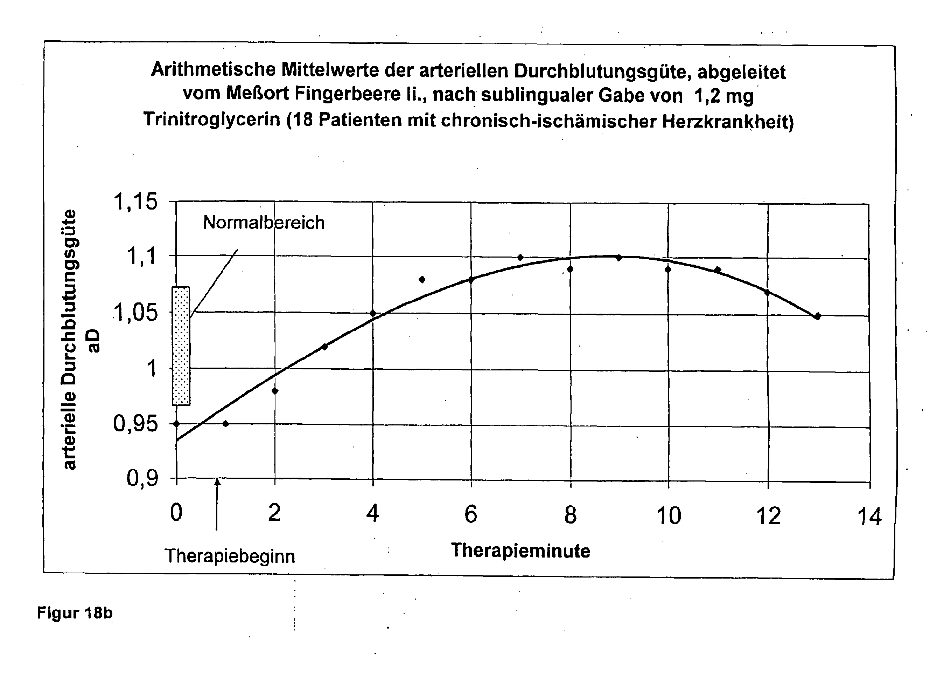

**Figur 18:** Quantitative Veränderungen von arteriellen Gefäßwandeigenschaften, speziell des Faser-Stretching FS (Figur 18 a) und der arteriellen Durchblutungsgüte aD (Figur 18 b), unter einem Funktionstest "sublinguale Gabe von 1,2 mg Trinitroglycerin" bei Patienten mit chronisch-ischämischer Herzkrankheit.

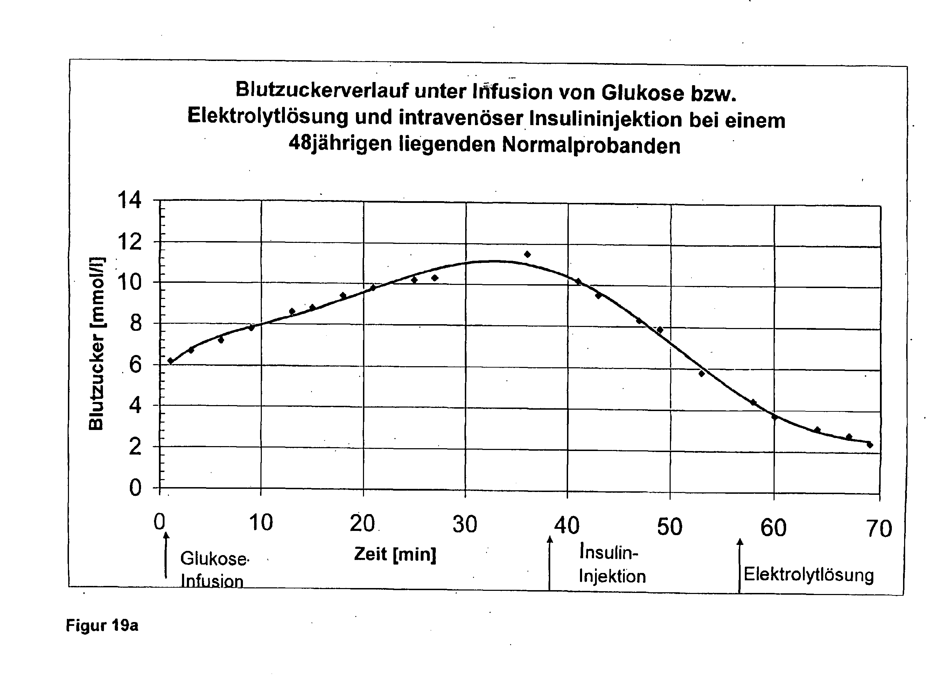

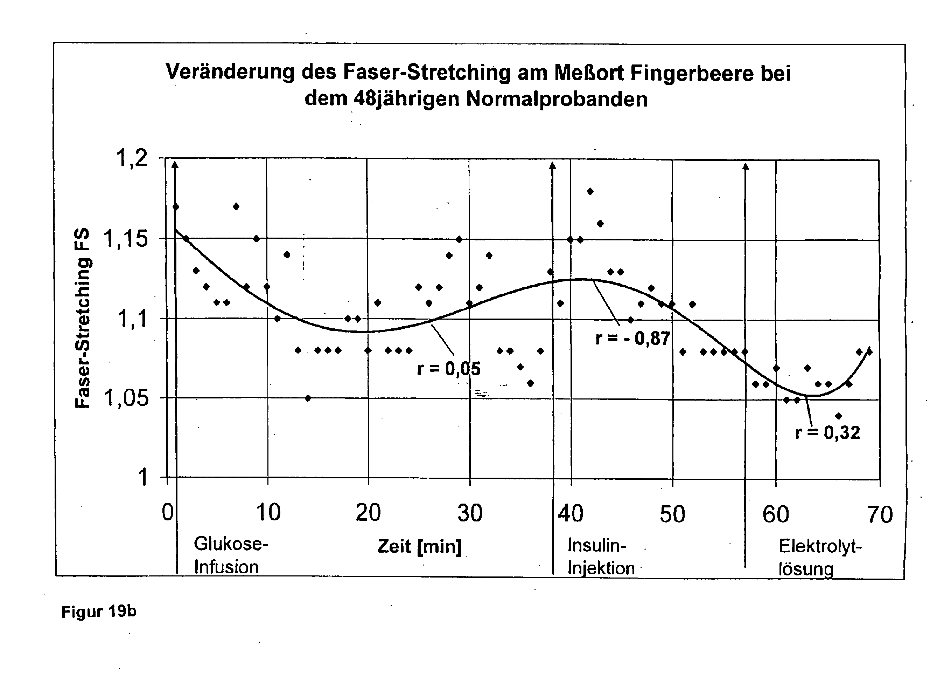

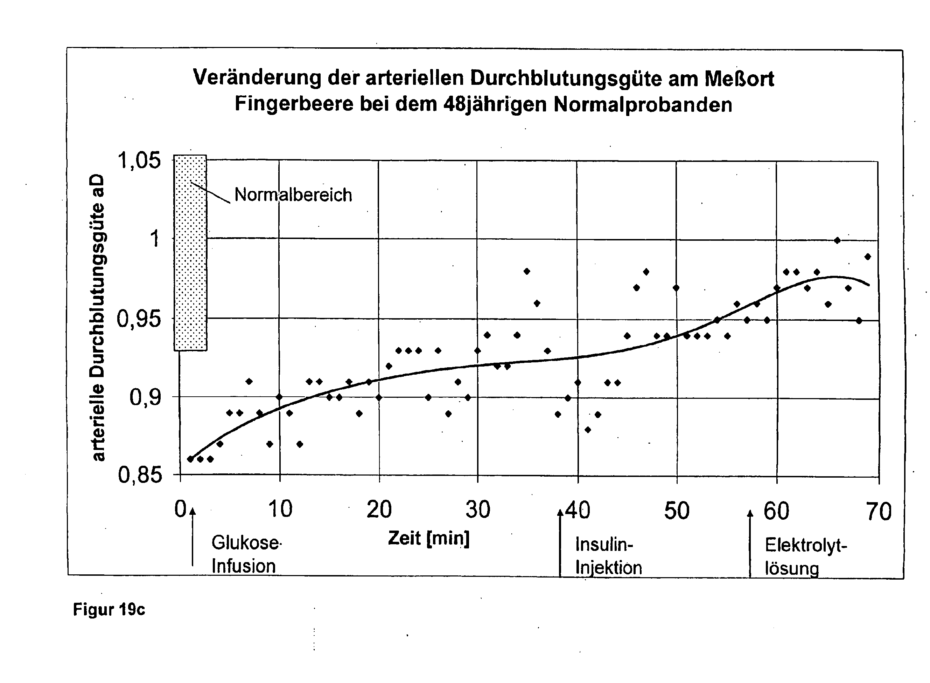

**Figur 19:** Veränderungen von Blutzucker, Faser-Stretching und arterieller Durchblutungsgüte, wie sie sich unter einem Funktionstest "intravenöse Applikation von Glukoselösung, Insulin und Elektrolytlösung" bei der 48jährigen Testperson (Arzt) am Messort Fingerbeere einstellten.

In Fig. 12 sind arterielle Durchblutungsgüte aD und Faser-Stretching FS bei 50 Patienten mit peripherer arterieller Verschlusskrankheit dargestellt, wobei die NIRP-Messungen vom Messort Großzehenbeere abgeleitet wurden. Die Mittelwerte aD = 0,68 sowie FS = 1,19 bestätigen die erfindungsgemäßen Darstellungen: verringerte arterielle Durchblutungsgüte, erhöhtes Faser-Stretching.

Figur 13 zeigt die Form der Blutvolumenpulsationen, abgeleitet vom Messort 2. Fingerbeere re. bei einem 27jährigen Probanden (Radsportprofi), wenn eine am Oberarm angelegte Kompression von 250 mm Hg plötzlich geöffnet wird. Unmittelbar nach Öffnung der Manschette kommt es zur Herausbildung der Pulsationen, wobei die Dikrotien zunächst relativ schwach ausgebildet sind, das zugehörige Faser-Stretching etwas erhöht ist, die arterielle Durchblutung an der unteren Grenze des Normalbereiches liegt. Bereits 16 Sekunden nach Öffnung sind die Dikrotien deutlicher ausgebildet, die Fläche unter den Pulsationen, also das mikrovaskuläre Blutfüllungsvolumen, nimmt zu, eine Gefäßdilatation ist eingetreten. Am Ende der 1. Minute nach Öffnung kommt es infolge der erhöhten Schubspannung des strömenden Blutes zur dominanten und anormalen Herausbildung der Dikrotien, also zu einem anormalen Rückgang der Dehnung der elastischen Fasern, die Gefäßdilatation ist weiter angestiegen. Der sich einstellende Übergangsprozess ist am Ende der 2. Minute nach Öffnung praktisch abgeschlossen. Da (die hier nicht dargestellte) pulsatile $O_2$-Sättigung sich deutlich reduzierte, kann auf eine anormal erhöhte NO-Freisetzung nach Öffnung der Kompression geschlossen werden. Es liegt keine endotheliale Dysfunktion vor.

Die Verläufe des mikrovaskulären Blutfüllungsvolumens und der pulsatilen arteriellen $O_2$-Sättigung, von Faser-Stretching und der Wendepunktzeit sowie von Form und Amplitude von Blutvolumenfluß und daraus abgeleiteter Blutvolumengeschwindigkeit bei einem 55jährigen Normalprobanden am Messort Fingerbeere sind in Figur 14 ersichtlich, wenn eine am Oberarm angelegte Kompression von 250 mm Hg plötzlich geöffnet wird. Aus den Verläufen in Figur 14a geht hervor, dass sich sofort nach Öffnung der Staumanschette das mikrovaskuläre Blutfüllungsvolumen kontinuierlich erhöht, gleichfalls die pulsatile arterielle $O_2$-Sättigung. Die durch die angelegte Kompression hervorgerufene Blutstauung muß über die Gefäßperipherie aufgelöst werden, was durch eine normale Gefäßweitung möglich ist. Nach etwa 30 Sekunden setzt eine verstärkte NO-Freisetzung durch die auf das Gefäßendothel wirkenden hohen Schubspannungen und eine nachfolgende NO-Oxidation ein, so dass als Normalverhalten neben der dadurch hervorgerufenen Gefäß-Dilatation eine Reduzierung der pulsatilen arteriellen $O_2$-Sättigung auftritt (z. B. in der 3. Minute nach Öffnung -17,6%). In Figur 14 b ist vor allem das Verhalten der Gefäßdehnung, des Faser-Stretching FS, ersichtlich. Unmittelbar nach Öffnung der Stauung erhöht sich FS auf das ca. 1,6-fache vom Normalwert (FS-Mittelwert der gesamten 1. Minute beträgt 1). In der 2. und 3. Minute reduziert sich FS in Form einer Übergangsfunktion beträchtlich (als jeweilige Mittelwerte auf 0,95). Die Auflösung der Blutstauung nach Öffnung der Staumanschette zeigt sich aus der Form der entsprechenden Blutvolumengeschwindigkeit, wie in Figur 14c dargestellt ist. Während in den ersten 4 Sekunden nach Öffnung dieser Übergangsprozeß vergleichbar ist mit einer (scheinbaren) "arteriellen Durchblutungsstörung" (vgl. mit Verlauf nach Figur 5 b) und sich ein reduzierter Mittelwert von aD = 0,93 für die 1. Minute ergibt, ändert sich die Form der Blutvolumengeschwindigkeit: am Ende der 1. Minute sowie der 3. Minute entspricht der Verlauf dem einer mediasklerotischen Gefäßveränderung (vgl. mit Figur 5 c), der Mittelwert der arteriellen Durchblutungsgüte erhöht sich in der 3. Minute auf aD = 0,98.

Figur 15 a, b und c zeigt charakteristische Verläufe des peripheren Faser-Stretching bei Normalpersonen und einem Typ II-Diabetiker, die unter jeweiliger Gabe von 40 g Traubenzucker als Funktions- bzw. Stimulationstest vom Messort Fingerbeere abgeleitet wurden.

Bei der 35jährigen Normalprobandin nach Figur 15 a ($HbA_{1c}$ = 5,3 %) bewirkt der nur relativ geringe Blutzuk-keranstieg, der sich unter Gabe von 40 g Traubenzucker einstellt, keine Erhöhung des im Normalbereich liegenden Faser-Stretching. Ermittelt man die Korrelation zwischen Faser-Stretching und den Blutzuckerwerten, so ergibt sich ein Wert von r = - 0,45. Eine Erhöhung der Blutzuckerwerte bewirkt eine Abnahme des Faser-Stretching, auch wenn diese Abhängigkeit gering ist. Der Blutdruck von 110/70 reduziert sich auf 105/60 mm Hg. Offensichtlich liegt hier ein intaktes Endothel vor.

Aus dem Verlauf nach Figur 15b bei einem 70jährigen "Normalprobanden" $HbA_{1c}$ = 5,2 %) mit 5 mmol/l Blutzucker *vor* Gabe von 40 g Traubenzucker und eigentlich nur relativ schwacher Erhöhung auf maximal 7,4 mmol/l, *erhöht* sich das Faser-Stretching nach etwa 20 Minuten, dabei ab einem Blutzuckergehalt von lediglich ca. 7 mmol/l. Dies könnte bei diesem Probanden eine *"Stabilitätsgrenze" des offensichtlich nicht mehr intakten Endothels* sein. Zwischen Faser-Stretching und Blutzucker besteht eine Korrelation von r = 0,5. Unter 40 g Traubenzucker stieg der Blutdruck von 150/85 auf 160/90 mm Hg. Dieser hinsichtlich des Zuckerstoffwechsels als "Normalproband" Einzustufende ist Herzschrittmacherpatient seit 1998 und ebenfalls Hypertoniker.

Bei dem 62jährigen Typ II-Diabetiker ($HbA_{1c}$ = 6,3 %) nach Figur 15c liegt offensichtlich eine Endotheldysfunktion vor: Das Faser-Stretching erhöht sich bis weit in den Nichtnormalbereich mit Erhöhung des Blutzuckers. Zwischen beiden tritt eine Korrelation von *r = 0,96* auf, also eine nahezu direkte Abhängigkeit. Der Blutdruck stieg unter Gabe von 40 g Traubenzucker von 150/90 auf 160/95 mm Hg. 6 Monate nach diesen Messungen trat eine absolute Arrythmie auf, nach 12 Monaten ein peripherer arterieller Verschluß im Oberschenkel, 2003 ein Schlaganfall. Das Sehvermögen verschlechterte sich zunehmend. Dem Patienten wurde vom Hausarzt nach obiger Messung empfohlen, täglich den systolischen und diastolischen Blutdruck sowie den Puls zu messen. Es liegen von diesem Patienten derartige Messungen kontinuierlich über einen Zeitraum von 200 Tagen vor, dabei früh, mittags und abends. Die Durchschnittswerte veränderten sich wie folgt: Blutdruck von 135/75 auf 155/80 mm Hg, Puls von 77 auf 90 min[-1].

Die Veränderungen der arteriellen Durchblutungsgüte aD, die sich unter Gabe von 40 g Traubenzucker bei den Versuchspersonen nach Figur 15 einstellten und vom Messort Fingerbeere abgeleitet wurden, zeigt Figur 16. Bei der 35jährigen Normalprobandin (Figur 16 a) ergeben sich unter diesem Funktionstest keine Reduzierungen, sondern es tritt ca. 35 Minuten nach Stimulation ein geringer Anstieg von aD ein. Zwischen aD und dem Blutzucker besteht mit r = 0,05 praktisch keine statistische Abhängigkeit, es liegt Normalverhalten des Gefäßendothels vor. Analog zum Faser-Stretching tritt bei dem 70jährigen Normalprobanden nach Figur 16b ein Stabilitätsgrenzfall auf: Mit steigendem Blutzucker erhöht sich als normale Gegenreaktion die zu Beginn verringerte arterielle Durchblutungsgüte, um nach ca. 20 Minuten abzufallen. Es besteht eine schwache negative Korrelation von r = - 0,15.

Eindeutig ist der Zusammenhang zwischen Blutzucker und aD mit r = - 0,8 bei dem 62jährigen Typ II-Diabetiker nach Figur 16 c: Mit steigendem Blutzucker reduziert sich die arterielle Durchblutungsgüte aD als Ausdruck der auftretenden endothelialen Dysfunktion.

Den Zusammenhang zwischen Faser-Stretching, abgeleitet vom peripheren Meßort Fingerbeere, sowie der Herzperiodendauer bei Normalpersonen und Typ II-Diabetikern nach den Figuren 15 und 16 unter Gabe von 40 g Traubenzucker zeigt Figur 17. Während bei den Normalpersonen der statistische Zusammenhang relativ schwach ist (Figur 17 a: r = 0,26; Figur 17 b: r = 0,14), steigt er bei dem 62jährigen Typ II-Diabetiker nach Figur 17c mit r = 0,77 deutlich an. Die endotheliale Dysfunktion ist offensichtlich nicht nur auf die Gefäßperipherie begrenzt. Aus den Darstellungen in Figur 17 geht des weiteren hervor, dass mit der nichtinvasiven NIRP-Methode auch das Zusammenwirken des Herzens mit der Gefäßperipherie bewertbar ist. Auf Grund der Progression der Endothelerkrankungen und der eingeschränkten therapeutischen Möglichkeiten in den Spätstadien entsteht die Forderung nach einer frühzeitigen Diagnose, die mit diesem Verfahren realisierbar ist.

Quantitative Veränderungen von arteriellen Gefäßwandeigenschaften, speziell des Faser-Stretching FS und der arteriellen Durchblutungsgüte aD, unter einem Funktionstest "sublinguale Gabe von 1,2 mg Trinitroglycerin" bei Patienten mit chronisch-ischämischer Herzkrankheit zeigt Figur 18. Bekanntlich stellt Nitroglycerin einen exogenen Stimulus dar, mit dem man die Vasodilatation endothelunabhängig anregen kann. Dieser Effekt läßt sich nichtinvasiv u. a. mit Hilfe hochauflösender Ultraschallduplexsonographie, der Laser-Doppler-Flowmetrie und der Venenverschlußplethysmographie erfassen. Ebenso ist der direkte Nachweis einer Akutwirkung invasiv, z. B. koronarangiographisch, möglich. Aus Figur 18 a und b geht hervor, dass mit der nichtinvasiven NIRP-Methode nicht nur die allgemeine Wirkung von Nitroglycerin erfassbar ist, sondern der Wirkungsverlauf ist als Übergangsfunktion quantitativ darstellbar. Hierfür wurden 18 Patienten mit chronisch-ischämischer Herzkrankheit, Schweregrad II - IV der NYHA-Klassifikation, in die Untersuchung einbezogen. Die Messungen erfolgten am liegenden Patienten an der Fingerbeere des 2. Fingers links. Nach einer Ausgangs- bzw. Bezugsmessung erhielten die Patienten jeweils 1,2 mg Trinitroglycerin-Spray (Nitro-Lingual®) sublingual. Die Registrierung der Pulsationskurven erfolgte kontinuierlich bis zu 15 Minuten. Speziell reduzierte sich unter sublingualer Gabe von 1,2 mg Trinitroglycerin das Faser-Stretching FS hochsignifikant (p ≤ 0,0004) von 1,01 $\pm$ 0,07 auf 0,94 $\pm$ 0,06, während sich die arterielle Durchblutungsgüte aD signifikant (p ≤ 0,05) bis zur 9. Minute erhöhte, um danach wieder bis zum Normalbereich abzufallen. Erwähnt sei auch, dass sich eine hochsignifikante Veränderung (p ≤ 0,01) der Puls(Herz)-Periodendauer von 830 $\pm$ 95 ms auf 811 $\pm$ 109 ms einstellte.

In Figur 19a, b und c sind Parameterveränderungen ersichtlich, wie sie sich unter einem Funktionstest "intravenöse Applikation von Glukoselösung, Insulin und Elektrolytlösung" ergaben. Speziell zeigt Figur 19a den Verlauf des Blutzuckers, wie er sich bei der 48jährigen Testperson (Arzt) unter intravenöser Applikation von Glukoselösung, Insulin und Elektrolytlösung einstellte. Unter der Infusion von 10%iger Glukoselösung mit einer Geschwindigkeit von 500 ml/h kommt es zu einem kontinuierlichen linearen Anstieg. Nach intravenöser Injektion von 15 I.E. Normalinsulin (Actrapid$^R$) ist ein deutlicher Rückgang unter den Ausgangswert zu verzeichnen. Die Blutzuckerwerte liegen im hypoglykämischen Bereich. Die Elektrolytlösung (E153) hat erwartungsgemäß keinen Einfluss auf den Blutzucker. Aus Figur 19b geht die Veränderung des Faser-Stretching (Meßort Fingerbeere) hervor. Während sich unter steigendem Blutglukosespiegel das Faser-Stretching als Gegenreaktion bis etwa zur 20. Minute reduziert, steigt es danach bis zum Ende der Glukose-Infusion wieder an. Dabei besteht praktisch keine Korrelation (r = 0,05) zwischen den beiden Größen. Deutlich ist der Abfall des Faser-Stretching nach der Insulin-Injektion ersichtlich (r = -0,87!). Ca. 25 Min. danach wird der Normalbereich des Faser-Stretching [0,94...1...1,06] erreicht. Die Veränderung der arteriellen Durchblutungsgüte aD am Meßort Fingerbeere, wie sie sich unter Infusion von Glukoselösung und Elektrolytlösung bzw. Insulin-injektion einstellen, geht aus Figur 19c hervor. Der kontinuierliche Anstieg von aD zeigt analog zum Faser-Stretching die Gegenreaktion dieser hämodynamischen Parameter beim Anstieg des Blutzuckers und erreicht am Ende der Glukose-Infusion den Normalbereich. Im Falle einer endothelialen Dysfunktion würde sich unter der Glukose-Infusion ein Abfall von aD einstellen.

Insgesamt zeigen diese Pilotuntersuchungen, dass es mit der NIRP-Methode möglich ist, die Wirkungen von physikalischen und medikamentösen Verfahren und Reizen auf die Hämodynamik einfach und schnell zu erfassen. Damit werden neben Funktionstestungen auch Dosisoptimierungen, auch unmittelbar am Krankenbett, möglich.

**Patentansprüche**

1. Arbeitsverfahren zum Betreiben einer Vorrichtung und Auswerten von Messergebnissen zur nichtinvasiven Ermittlung hämodynamischer Funktionen des Blutgefäßsystems, einschließlich des Endothels, wobei die Vorrichtung umfasst:

   - Mittel zum Anlegen einer Blutdruck-Staumanschette an den Extremitäten eines Individuums, z. B. Ober-/ Unterarm, Ober-/Unterschenkel, Vorder-/ Hinterlauf und gleichzeitige nichtinvasive Ableitung der an der entsprechenden Gefäßperipherie sich einstellenden mikrovaskulären Blutvolumenpulsationen, um daraus funktionelle Blutgefäßwandparameter zu bestimmen,
   - Mittel zur Erzeugung einer Stauung bis zu einem Kompressionsdruck, bei dem die an der Gefäßperipherie dieser Extremität abgeleiteten Blutvolumenpulsationen nicht mehr ableitbar sind,
- Mittel zum sprungartigen Öffnen der Kompression nach einer Stauzeit von 5 ... 10 Minuten, so dass die peripheren Blutgefäß-Kennfunktionen sich als Sprungübergangsfunktionen gemäß der Systemtheorie abbilden und aus deren zeitlichen Verläufen der hämodynamische Zustand ermittelt wird sowie
- Mittel für allgemeine Funktionstests, die zu Veränderungen vor allem der Schubspannung des strömenden Blutes auf eine Gefäßwand führen und damit durch spezielle externe Verfahren bzw. Stimuli, auch unter Pharmaka, gezielt Einfluß auf eine Freisetzung des Vermittlers einer endothelvermittelten Kreislaufregulation, von Stickstoffmonoxid (NO), genommen wird,

**dadurch gekennzeichnet,**
**dass** die zur Messung der peripheren Blutgefäßwand-Parameter zugrunde gelegte Zwei-Wellenlängen-NIR-ROT-Remissions-Photoplethysmographie einschließlich Zwei-Wellenlängen-NIR-ROT-Transmissions-Photoplethysmographie mit den Wellenlängen 840...940 nm (isosbestscher Bereich) und 640 nm um eine Wellenlänge im Bereich von ca. 390...405 bzw. 415...430 nm erweitert wird, um damit umfassende Mittel zur Beschreibung der endothelialen Funktion in die Vorrichtung zu integrieren, insbesondere

• über eine Wellenlänge im Bereich von ca. 390...405 bzw. 415...430 nm das Verhalten von Stickstoffmonoxid bezüglich des Blutvolumens (840 ... 940 nm) sowie dessen Oxygenierungszustandes (640 nm) zu erfassen,
• Formähnlichkeitsmaße (Korrelationsfaktoren) je Herzaktion (Pulsation) n zu ermitteln, dabei für eine bestimmte Messzeit Mittelwert und Standardabweichung, speziell
zwischen den Wellenlängen 840 nm und ca. 390...405 bzw. 415...430 nm : peripherer Stickstoffmonoxid(NO)-induzierter Koeffizient $pNOC_1$ sowie zwischen den Wellenlängen 640 nm und ca. 390...405 bzw. 415...430 nm : peripherer Stickstoffmonoxid(NO)-induzierter Koeffizient $pNOC_2$, zu erhalten,
• Zeitmaße $\tau_1$, $\tau_2$ und $\tau_3$ als Differenz der Gipfelzeiten $T_G$ der Pulsationen bei ca. 390...405 bzw. 415...430 nm sowie 840 ... 940 nm zu bestimmen, analog der entsprechenden Wendepunktzeiten $T_W$ der Blutvolumen-geschwindigkeiten sowie der Beschleunigungszeiten $T_{BE}$ der Blutvolumenbeschleunigung, wobei
• Algorithmen zur Bildung der Füllungsvolumina für 840 und 640 nm auf diejenigen bei einer Wellenlänge aus dem Bereich von ca. 390...405 bzw. 415...430 nm erweitert werden,

wobei bei der Messung von Maßen zur Beschreibung der endothelialen Funktion, wie Gipfelzeiten $T_G$, Füllungsvolumina und Formähnlichkeitsmaße, mittels dreier Wellenlängen, die jeweiligen Maße für jede Wellenlänge abgeleitet und miteinander in Relation gesetzt werden.

2.  Arbeitsverfahren nach Anspruch 1,
    **dadurch gekennzeichnet,**
    **dass** zur nichtinvasiven Bestimmung von funktionellen Gefäßwandeigenschaften aus den bei der Wellenlänge 840 ... 940 nm sich einstellenden mikrovaskulären Blutvolumenpulsationen die mathematische 1. und 2. Ableitung (Blutvolumengeschwindigkeit, Blutvolumenbeschleunigung) mit charakteristischen Zeiten, Zeit- und Flächenquotienten gebildet werden, wobei aus dem Verlauf der Blutvolumengeschwindigkeit einer Pulsperiode die Wendepunktzeit $T_W$ als die Zeit, wo das Geschwindigkeitsmaximum auftritt und
    das Flächenverhältnis von rechter und linker Fläche $F_1$ / $F_2$ zur Wendepunktzeit liegend, ein Dehnungsmaß für die elastischen Fasern in der Gefäßwand darstellt und das Dehnungsmaß mit ausreichender Näherung auch mit dem diesen Flächen zugehörigen Zeitverhältnis $T_B$ / $T_W$ gleichsetzbar ist.

3.  Arbeitsverfahren nach Anspruch 1 oder 2,
    **dadurch gekennzeichnet,**
    **dass** aus den aus der Blutvolumengeschwindigkeit ableitbaren peripheren Gefäßwandeigenschaften Dehnungsmaß (FS) und Wendepunktzeit ($T_W$) eine arterielle Durchblutungsgüte aD bestimmbar ist, wobei allgemein gilt

$$aD \approx \frac{1}{FS \bullet T_W}$$

4.  Arbeitsverfahren nach den Ansprüchen 2 oder 3,
    **dadurch gekennzeichnet,**
    **dass** zur umfassenden Bestimmung der hämodynamischen Gefäßwandeigenschaften einschließlich Endothel bei Durchführung eines Funktionstests neben den Formähnlichkeitsmaßen $pNOC_1(n)$ und $pNOC_2(n)$ und den Zeitma-

ßen $\tau_1$, $\tau_2$ und $\tau_3$ auch die Veränderungen des mikrovaskulären Blutfüllungsvolumens, der arteriellen Durchblutungsgüte aD, des Dehnungsmaßes (FS) sowie der pulsatilen $O_2$-Sättigung integriert werden, die nichtinvasiv mittels Photoplethysmographie bei den Wellenlängen 840...940 nm und 640 nm bestimmbar sind.

**Claims**

1. Working method for operating a device and for analyzing measuring results for the non-invasive detection of hemodynamic functions of the blood vessel system, including the endothelium, said device comprising:

   - means for applying a blood pressure tourniquet cuff to the extremities of an individual, e.g. the upper arm, forearm, upper/lower leg, foreleg, hind leg, and simultaneous non-invasive derivation of the microvascular blood volume pulsations occurring at the respective vessel periphery so as to determine therefrom functional blood vessel wall parameters,
   - means for creating a congestion until a compression pressure at which the blood volume pulsations derived at the vessel periphery of this extremity are no longer derivable,
   - means for abruptly opening the compression after a congestion period of 5 ... 10 minutes, so that the characteristic functions of the peripheral blood vessel are represented as step-function responses according to the systems theory and the hemodynamic state is determined from the variations with time thereof, and
   - means for general function tests which lead to changes above all of the shearing stress of the flowing blood on a vascular wall and wherein, by means of special external methods or stimuli, also under medication, a release of the mediator of an endothelium-mediated circulation regulation, of nitrogen monoxide (NO), is selectively influenced,

   **characterized in that**
   the dual wavelength NIR-ROT remission photoplethysmography on which the measurement of the peripheral blood vessel wall parameters is based, including dual wavelength NIR-ROT transmission photoplethysmography with the wavelengths 840...940 nm (isosbestic range) and 640 nm, is extended by a wavelength in the range of about 390... 405 or 415...430 nm so as to integrate in the device comprehensive means for the description of the endothelial function, specifically,

   • detecting by a wavelength in the range of about 390...405 or 415... 430 nm the behavior of nitrogen monoxide with respect to the blood volume (840 ... 940 nm) and the oxygenation condition (640 nm) thereof,
   • determining shape similarity quantities (correlation factors) per heartbeat (pulsation) n, thereby obtaining a mean value and a standard deviation for a certain time of measurement, specifically
   between the wavelengths 840 nm and about 390...405 or 415...430 nm: peripheral nitrogen monoxide (NO) induced coefficient $pNOC_1$ and
   between the wavelengths 640 nm and about 390...405 or 415...430 nm: peripheral nitrogen monoxide (NO) induced coefficient $pNOC_2$ ,
   • determining time scales $\tau_1$, $\tau_2$ and $\tau_3$ as difference of the peak times $T_G$ of the pulsations at about 390...405 or 415...430 nm and 840 ... 940 nm, analogously to the corresponding turning point times $T_W$ of the blood volume velocities and the acceleration times $T_{BE}$ of the blood volume acceleration, wherein
   • algorithms for determining filling volumes for 840 and 640 nm are extended to those at a wavelength from the range of about 390...405 or 415...430 nm, wherein the respective quantities for each wavelength are derived and brought into a relation with each other by means of three wavelengths during the measurement of quantities for the description of the endothelial function, such as peak times $T_G$, filling volumes and shape similarity quantities.

2. Working method according to claim 1,
   **characterized in that**
   for the non-invasive determination of functional vessel wall properties from the microvascular blood volume pulsations occurring at the wavelength 840 ... 940 nm the mathematical 1. and 2. derivative (blood volume velocity, blood volume acceleration) are obtained with characteristic times, time and surface quotients, wherein the turning point time $T_W$ is determined from the behavior of the blood volume velocity of a pulse period as the time when the velocity maximum occurs, and
   the surface ratio of the right and left surface $F_1 / F_2$ at the turning point time represents an expansion quantity for the elastic fibers in the vessel wall and the expansion quantity can be equated with sufficient approximation also to the time ratio $T_B / T_W$ associated with these surfaces.

3.  Working method according to claim 1 or 2,
    **characterized in that**
    from the peripheral vessel wall properties expansion quantity (FS) and turning point time ($T_W$) derivable from the blood volume velocity an arterial blood flow quality aD is determinable according to

$$aD \approx \frac{1}{FS \bullet T_W}$$

4.  Working method according to claims 2 or 3,
    **characterized in that**
    for the comprehensive determination of the hemodynamic vessel wall properties, including the endothelium, during the performance of a function test not only the shape similarity quantities $pNOC_1(n)$ and $pNOC_2(n)$ and the time scales $\tau_1$, $\tau_2$ and $\tau_3$ , but also the changes of the microvascular blood filling volume, of the arterial blood flow quality aD, of the expansion quantity (FS) and of the pulsatile $O_2$-saturation are integrated, which can be determined non-invasively by means of photoplethysmography at the wavelengths 840...940 nm and 640 nm.

**Revendications**

1.  Procédé de travail destiné à exploiter un dispositif et à évaluer des résultats de mesure pour le dépistage non invasif de fonctions hémodynamiques du système vasculaire, y compris celles de l'endothélium, le dispositif comportant :

    - des moyens de poser un brassard de compression artérielle aux extrémités d'un individu, par ex. bras / avant-bras, cuisse / jambe, membre inférieur / membre postérieur, et dérivation simultanée non invasive des pulsations microvasculaires de volume sanguin qui s'établissent à la périphérie vasculaire correspondante afin d'en déduire des paramètres fonctionnels de paroi vasculaire,
    - des moyens de générer une congestion jusqu'à une pression de compression à laquelle les pulsations de volume sanguin dérivées à la périphérie vasculaire de cette extrémité ne sont plus dérivables,
    - des moyens d'ouvrir par à-coup la compression après un temps de congestion de 5 ... 10 minutes de sorte que les fonctions périphériques caractéristiques de vaisseau sanguin se présentent comme des fonctions de transition par à-coup conformément à la théorie des systèmes et que l'état hémodynamique soit déterminé à partir de leurs allures temporelles, ainsi que
    - des moyens de réaliser des tests fonctionnels généraux qui entraînent des modifications notamment de la tension de poussée du flux sanguin sur une paroi vasculaire et permettent ainsi d'avoir, par des procédés ou stimuli externes spéciaux, y compris sous pharmacopée, une influence ciblée sur une libération du médiateur d'une régulation circulatoire à médiation endothéliale, le monoxyde d'azote (NO),

    **caractérisé en ce que**
    la photopléthysmographie de rémission NIR-ROT à deux longueurs d'onde servant de base à la mesure des paramètres périphériques de paroi vasculaire, y compris la photopléthysmographie de transmission NIR-ROT à deux longueurs d'onde, avec les longueurs d'onde de 840...940 nm (plage isosbésique) et de 640 nm, est complétée d'une longueur d'onde de l'ordre d'env. 390...405 ou 415...430 nm afin d'intégrer ainsi dans le dispositif de vastes moyens pour la description de la fonction endothéliale, en particulier

    - relever, via une longueur d'onde de l'ordre d'env. 390...405 ou 415...430 nm, le comportement du monoxyde d'azote eu égard au volume sanguin (840...940 nm) ainsi que son état d'oxygénation (640 nm),
    - déterminer des grandeurs de similitude de forme (facteurs de corrélation) par action cardiaque (pulsation) n, et obtenir à cette occasion, pour un temps de mesure défini, une valeur moyenne et un écart standard, spécialement
    entre les longueurs d'onde de 840 nm et d'env. 390...405 ou 415...430 nm : coefficient périphérique induit par le monoxyde d'azote (NO) $pNOC_1$ ainsi que entre les longueurs d'onde de 640 nm et d'env. 390...405 ou 415...430 nm : coefficient périphérique induit par le monoxyde d'azote (NO) $pNOC_2$,
    - déterminer des grandeurs de temps $\tau_1$, $\tau_2$ et $\tau_3$ comme différence des temps de crête $T_G$ des pulsations à env. 390...405 ou 415...430 nm ainsi qu'à 840...940 nm, par analogie aux temps correspondants de point de retour $T_W$ des vitesses de volume sanguin ainsi qu'aux temps d'accélération $T_{BE}$ de l'accélération de volume sanguin,

- des algorithmes de formation des volumes de remplissage pour 840 et 640 nm étant étendus à ceux pour une longueur d'onde comprise dans la plage d'env. 390...405 ou 415...430 nm,

les grandeurs respectives pour chaque longueur d'onde étant dérivées et mises en relation entre elles moyennant trois longueurs d'onde lors de la mesure de grandeurs pour la description de la fonction endothéliale, comme des temps de crête $T_G$, des volumes de remplissage et des grandeurs de similitude de forme.

2. Procédé de travail selon la revendication 1,
   **caractérisé en ce que**
   pour la détermination non invasive de propriétés fonctionnelles de paroi vasculaire à partir des pulsations microvasculaires de volume sanguin qui s'établissent à la longueur d'onde de 840...940 mm, la 1e et 2e dérivée mathématique (vitesse de volume sanguin, accélération de volume sanguin) sont formées avec des temps caractéristiques, des quotients de temps et de surface, le temps de point de retour $T_w$ étant le temps où le maximum de vitesse se produit dans l'allure de la vitesse de volume sanguin d'une période de pulsation et

   le rapport de surface des surfaces droite et gauche $F_1 / F_2$ par rapport au temps de point de retour représentant une grandeur d'allongement pour les fibres élastiques dans la paroi vasculaire et la grandeur d'allongement étant aussi assimilable avec une approximation suffisante à ce rapport de temps $T_B / T_w$ associé à ces surfaces.

3. Procédé de travail selon la revendication 1 ou 2,
   **caractérisé en ce que**
   à partir des propriétés périphériques de paroi vasculaire dérivables de la vitesse de volume sanguin que sont la grandeur d'allongement (FS) et le temps de point de retour ($T_W$), une qualité de circulation artérielle aD soit déterminable, étant posé de manière générale que :

$$aD \approx \frac{l}{FS \bullet T_W}$$

4. Procédé de travail selon les revendications 2 ou 3,
   **caractérisé en ce que**
   pour la détermination étendue des propriétés hémodynamiques de paroi vasculaire, y compris l'endothélium, les modifications du volume de remplissage sanguin microvasculaire, de la qualité de circulation artérielle aD, de la grandeur d'allongement (FS) ainsi que de la saturation pulsatile en $O_2$ sont, lors de l'exécution d'un test fonctionnel, intégrées en plus des grandeurs de similitude de forme $pNOC_1(n)$ et $pNOC_2(n)$ et des grandeurs de temps $\tau_1$, $\tau_2$ et $\tau_3$, lesquelles modifications peuvent être déterminées de manière non invasive par photopléthysmographie aux longueurs d'onde de 840...940 nm et de 640 nm.

**Figur 1**

**Figur 2**

**Figur 3**

Figur 4

Figur 5

EP 1 584 289 B1

Abhängigkeit von RPW$_2$ vom Faser-Stretching FS

r = 0,8

Faser-Stretching FS

Relativer Peripherer Widerstand RPW$_2$ [%]

Figur 6

24

Zusammenhang zwischen dem Zeitquotienten $T_B/T_W=(T_G-T_W)/T_W$ und dem Faser-Stretching FS

Figur 7

EP 1 584 289 B1

Zusammenhang zwischen der Wendepunktzeit $T_W$ und dem Faser-Stretching FS bei Versuchspersonen nach Figur 5 mit ausgebildeten Dikrotien

Figur 8

EP 1 584 289 B1

**Zusammenhang Faser-Stretching und Wendepunktzeit bei pAVK-Patienten am Meßort Großzehenbeere**

Figur 9

EP 1 584 289 B1

Figur 10

EP 1 584 289 B1

**Figur 11**

Figur 12a

EP 1 584 289 B1

arterielle Durchblutungsgüte aD bei pAVK-Patienten

Figur 12b

EP 1 584 289 B1

Figur 13

## 1. Minute

Blutvolumen / Änderung Oxygenierung      Manschette, Blutdruck

Manschette, Blutdruck; MESS0036.70S; 4.7.2003, 8:26Uhr   T:32.0°C;

Bezugsmessung MESS0036

Veränderung $\Delta O_2$ der Oxygenierung (pulsatiles Signal) [%]

mikrovaskuläres (pulsatiles) Blutfüllungsvolumen mBFV [Volumen]

**Beginn der Reduzierung der pulsatilen arteriellen O₂-Sättigung**

**Öffnung der Staumanschette**

**mikrovaskuläres Blutfüllungsvolumen**

**pulsatile arterielle O₂-Sättigung**

0 3 6 9 12 15 18 21 24 27 30 33 36 39 42 45 48 51 54 57 60 63 66 69 72 75

Kanal 1: –+–+–+– Mittelwert Blutvolumen = 1.00 Volumen      Pulsnummer n
Blutvolumenvariabilität = 29.5 % [Normalbereich 8% ... 13% ... 18%]
Mittelwert Pulsperiodik / Blutfüllungsvolumen = 0.9 Sekunden / Volumen
(Bezugsmessung = 0.9 Sekunden / Volumen)
Kanal 1,2: ·+·+·+·· Mittelwert Oxygenierungs-Änderung = 0.0 %
Oxygenierungsvariabilität = 10.1 %

Figur 14 a / Blatt 1

## 2. Minute

Blutvolumen / Änderung Oxygenierung                    Manschette,Blutdruck

| Manschette, Bluldruck; MESS0037.70S; 4.7.2003, 8:27Uhr | T:32.5°C; |

Veränderung   mikrovaskuläres                              Bezugsmessung MESS0036
dO₂ der Oxy-  (pulsatiles)
genierung     Blutfüllungs-
(pulsatiles   volumen mBFV
Signal)       (Volumen)
[%]

mikrovaskuläres
Blutfüllungsvolumen

mittlere Veränderung der
pulsatilen arteriellen O₂-Sättigung
in der 2. Minute nach Öffnung = - 9,3 %

0  3  6  9  12 15 18 21 24 27 30 33 36 39 42 45 48 51 54 57 60 63 66 69 72

Kanal 1: ─+++++─ Mittelwert Blutvolumen = 1.20 Volumen            Pulsnummer n
         Blutvolumenvariabilität = 6.3 % [Normalbereich: 8% ... 13% ... 18%]
         Mittelwert Pulsperiodik / Blutfüllungsvolumen = 0.7 Sekunden / Volumen
         (Bezugsmessung = 0.9 Sekunden / Volumen)
Kanal 1,2: ·+++++· Mittelwert Oxygenierungs-Änderung = -9.3 %
         Oxygenierungsvariabilität = 1.5 %

Figur 14 a / Blatt 2

3. Minute

Blutvolumen / Änderung Oxygenierung          Manschette, Blutdruck

Manschette, Blutdruck; MESS0038.70S; 4.7.2003, 8:28Uhr | T:33.0°C;

Veränderung mikrovaskuläres                           Bezugsmessung: MESS0036
$\Delta O_2$ der Oxy- (pulsatiles)
genierung Blutfüllungs-
(pulsatiles volumen aBFV
Signal) (Volumen)
[%]

mikrovaskuläres
Blutfüllungsvolumen

pulsatile arterielle
$O_2$-Sättigung

mittlere Veränderung der
pulsatilen arteriellen $O_2$-Sättigung
in der 3. Minute nach Öffnung = - 17,6 %

0 3 6 9 12 15 18 21 24 27 30 33 36 39 42 45 48 51 54 57 60 63 66 69 72 75

Kanal 1: —+++++— Mittelwert Blutvolumen = 1.16 Volumen          Pulsnummer n
Blutvolumenvariabilität = 6.7 % (Normalbereich: 8% ... 13% ... 18%)
Mittelwert Pulsperiodik / Blutfüllungsvolumen = 0.7 Sekunden / Volumen
(Bezugsmessung = 0.9 Sekunden / Volumen)
Kanal 1,2: -+++- Mittelwert Oxygenierungs-Änderung = -17.6 %
Oxygenierungsvariabilität = 3.1 %

Figur 14 a / Blatt 3

## 1. Minute

Wendepunktzeit TW, Faser-Stretching FS        Manschette, Blutdruck

Manschette, Blutdruck: MESS0036.70S; 4.7.2003, 8:26Uhr | T:32.0°C:

$T_W$[ms]   FS

arterielle Durchblutungsgüte $aD = \frac{101}{FS \cdot TW} = 0.93$        N: 0,94...1...1,06

mikrovaskuläre Durchblutungsgüte $mD = \frac{1}{T_W \cdot FU} = 0.79$        N: 0,69...1...1,82

**erhöhtes Faser-Stretching unmittelbar nach Öffnung der Stauung (Rückgang von FS in Form einer Übergangsfunktion)**

H(FS)

H($T_W$)

**Verlauf der Wendepunktzeit**

**Öffnung der Staumanschette**

N...Normalbereiche

0  3  6  9  12 15 18 21 24 27 30 33 36 39 42 45 48 51 54 57 60 63 66 69 72 75

Pulsnummer n

Pulsquotient $\overline{PQ} = 0.37$        Wendepunkt-Zeitquotient $\overline{TQ} = 0.18$        $r_{TW,FS} = -0.61$  $r_{FU;FS} = -0.61$

Gipfelzeit $\overline{T_G} = 223$ ms        Wendepunktzeit $\overline{T_W} = 109$ ms        Faser-Stretching $\overline{FS} = 1.00$

Figur 14 b / Blatt 1

## 2. Minute

Wendepunktzeit TW, Faser-Stretching FS          Manschette, Blutdruck

Manschette, Blutdruck; MESS0037.70S; 4.7.2003, 8:27Uhr   T:32.5°C:

$T_{n}[ms]$  FS

arterielle Durchblutungsgüte $aD = \dfrac{101}{FS*TW} = 0.97$          N: 0,94...1...1,06

mikrovaskuläre Durchblutungsgüte $mD = \dfrac{1}{N_{LFV}} = 4.39$     N: 0,68...1...1,82

**Faser-Stretching**

N(FS)

N(T$_\mu$)

**Verlauf der Wendepunktzeit**

N...Normalbereiche

0  3  6  9  12  15  18  21  24  27  30  33  36  39  42  45  48  51  54  57  60  63  66  69  72

Pulsnummer n

Pulsquotient $\overline{PQ} = 0.33$          Wendepunkt-Zeitquotient $\overline{TQ} = 0.17$     $r_{TW,FS} = -0.49$  $r_{FVl;FS} = 0.10$

Gipfelzeit $\overline{T_G} = 212$ ms          Wendepunktzeit $\overline{T_W} = 109$ ms     Faser-Stretching $\overline{FS} = 0.95$

Figur 14 b / Blatt 2

# 3. Minute

Wendepunktzeit TW, Faser-Stretching FS          Manschette, Blutdruck

| Manschette, Blutdruck: MESS0038.70S; 4.7.2003, 8:28Uhr | T:33.0°C; |

$T_W$[ms]  FS

arterielle Durchblutungsgüte $aD = \dfrac{101}{FS \times TW} = 0.98$          N: 0,94...1...1,06

mikrovaskuläre Durchblutungsgüte $mD = \dfrac{1}{n_{LFV}} = 4.95$          N: 0,69...1...1,82

**Faser-Stretching**

N(FS)

**Verlauf der Wendepunktzeit**

N($T_W$)

N...Normalbereiche

0  3  6  9  12 15 18 21 24 27 30 33 36 39 42 45 48 51 54 57 60 63 66 69 72 75

Pulsnummer n

Pulsquotient $\overline{PQ} = 0.34$     Wendepunkt-Zeitquotient $\overline{TQ} = 0.17$     $r_{TW,FS} = -0.45$  $r_{FVI;FS} = -0.12$

Gipfelzeit $\overline{T_G} = 211$ ms     Wendepunktzeit $\overline{T_W} = 109$ ms     Faser-Stretching $\overline{FS} = 0.95$

Figur 14 b / Blatt 3

Figur 14 c

Figur 15a

EP 1 584 289 B1

Faser-Stretching am Meßort Fingerbeere bei einem 70jährigen liegenden Normalprobanden unter 40 g Traubenzucker

Zusammenhang Faser-Stretching und Blutzucker r = 0,5

Figur 15b

**Faser-Stretching am Meßort Fingerbeere bei einem 62jährigen liegenden Typ II-Diabetiker unter 40 g Traubenzucker**

Zusammenhang Faser-Stretching und Blutzucker r = 0,96

Zeit [min]

**Figur 15c**

EP 1 584 289 B1

arterielle Durchblutungsgüte am Meßort Fingerbeere bei einer 35jährigen liegenden Normalprobandin unter 40 g Traubenzucker

Figur 16a

EP 1 584 289 B1

**arterielle Durchblutungsgüte am Meßort Fingerbeere bei einem 70jährigen liegenden Normalprobanden unter 40 g Traubenzucker**

EP 1 584 289 B1

Figur 16b

Figur 16c

Zusammenhang von Faser-Stretching am Meßort Fingerbeere und Herzperiodendauer bei einer 35jährigen Normalprobandin unter 40 g Traubenzucker

r = 0,26

Faser-Stretching FS

Herzperiodendauer [ms]

Figur 17a

**Zusammenhang zwischen Faser-Stretching am Meßort Fingerbeere und Herzperiodendauer bei einem 70jährigen liegenden Normalprobanden unter 40 g Traubenzucker**

Figur 17b

EP 1 584 289 B1

**Zusammenhang zwischen Faser-Stretching am Meßort Fingerbeere und Herzperiodendauer bei einem 62jährigen liegenden Typ II-Diabetiker unter 40 g Traubenzucker**

r = 0,77

Faser-Stretching FS

Herzperiodendauer $T_H$ [ms]

Figur 17c

EP 1 584 289 B1

**Arithmetische Mittelwerte des Faser-Stretching am Meßort Fingerbeere li. nach sublingualer Gabe von 1,2 mg Trinitroglycerin (18 Patienten mit chronisch-ischämischer Herzkrankheit)**

Normalbereich FS: 0,94 ... 1,06

Faser-Stretching FS

0Therapie-beginn

Therapieminute

Figur 18a

EP 1 584 289 B1

**Arithmetische Mittelwerte der arteriellen Durchblutungsgüte, abgeleitet vom Meßort Fingerbeere li., nach sublingualer Gabe von 1,2 mg Trinitroglycerin (18 Patienten mit chronisch-ischämischer Herzkrankheit)**

Figur 18b

**Blutzuckerverlauf unter Infusion von Glukose bzw. Elektrolytlösung und intravenöser Insulininjektion bei einem 48jährigen liegenden Normalprobanden**

Figur 19a

EP 1 584 289 B1

**Veränderung des Faser-Stretching am Meßort Fingerbeere bei dem 48jährigen Normalprobanden**

Figur 19b

EP 1 584 289 B1

## Veränderung der arteriellen Durchblutungsgüte am Meßort Fingerbeere bei dem 48jährigen Normalprobanden

**Figur 19c**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 02314105 A2
- EP 97118971 A
- DE 4238641
- DE P4322860735
- WO 0234105 A2

### In der Beschreibung aufgeführte Nicht-Patentliteratur

- **Drexler H ; Hornig B.** Endothelial dysfunction in human disease. *J Mol Cell Cardiol.,* Januar 1999, vol. 31 (1), 51-60
- **Beinder, E. ; W. Frobenius.** Die Präeklampsie: Eine Endothelerkrankung. *Deutsches Ärzteblatt Jg. 97,* 13. Oktober 2000
- **Celermajer DS ; Sorensen KE ; Gooch VM ; Spiegelhalter DJ ; Miller OI ; Sullivan ID ; Lloyd JK ; Deanfield JE.** Non-invasive detection of endothelial dysfunction in children and adults at risk of atherosclerosis. *Lancet,* 1992, vol. 340 (8828), 1111-5
- **Celermajer DS.** Testing endothelial function using ultrasound. *J Cardiovasc Pharmacol.,* 1998, vol. 32 (3), 29-32
- **Sorensen KE ; Celermajer DS ; Spiegelhalter DJ ; Georgakopoulos D ; Robinson J ; Thomas O ; Deanfield JE.** Non-invasive measurement of human endothelium dependent arterial responses: accuracy and reproducibility. *Br Heart J.,* 1995, vol. 74, 247-53
- **Furchgott, R.F. ; J. V. Zawadski.** The obligatory role of endothelial cells in the relaxation of arterial smooth muscle by acetylcholine. *Nature,* 1980, vol. 288, 373-376
- NO. Mittler, Missetäter und Medikament. **Scriba, P. C. ; S. Endres.** Sonderheft Internist. Springer, 1997, vol. 38, 405
- **Schmidt, H.H.H.W.** NO, endogener Botenstoff und Zellgift. *MED. MO. PHARM.,* 1994, vol. 17 (6), 168-185
- **Kelm, M.** *Kardiovaskuläre Wirkungen von Stickstoffmonoxid und ihre Bedeutung für die arterielle Hypertonie,* 1996
- Guidelines for the ultrasound assessment of endothelial-dependent flow-mediated vasodilation of the brachial artery: a report of the International Brachial Artery Reactivity Task Force. **Corretti MC ; Anderson TJ ; Benjamin EJ ; Celermajer D ; Charbonneau F ; Creager MA ; Deanfield J ; Drexler H ; Gerhard-Herman M ; Herrington D.** J Am Coll Cardiol. 2002, vol. 39, 257-65
- **Thews, G. ; P. Vaupel.** Vegetative Physiologie. Springer, 1997, vol. 3
- **Burton, A.C.** Physiologie und Biophysik des Kreislaufs. F.K. Schättauer, 1969
- **Kelm, M.** Kardiovaskuläre Wirkungen von Stickstoffmonoxid und ihre Bedeutung für die arterielle Hypertonie. Schattauer, 1996
- **KrauB, M. ; E.-G. Woschni.** Meßinformationssysteme. Kennfunktionen, Gütekriterien, Optimierung. VEB Verlag Technik, 1975
- **Krauß, M ; G. Grohmann ; J. Addae ; Ch. Posthoff ; R. Saunders.** *Non-invasive Recording of Parameters of the Heart*
- **Thews, G. ; P. Vaupel.** Vegetative Physiologie. Springer, 1997
- **Ritz Ritz E.** Exempla hypertonica: Bildatlas zur Pathophysiologie und Pharmakologie der Hypertonie. *München: Med. Service,* 1990, vol. 2
- **Schmid-Schönbein, H ; Grunau, G ; H Bräuer.** Exempla hämorheologica. Albert-Roussel Pharma GmbH, 1980
- Meßinformationssysteme. **Krauß, M. ; E.-G. Woschni.** Kennfunktionen, Gütekriterien, Optimierung. VEB Verlag Technik, 1975